(19) Europäisches Patentamt
European Patent Office
Office européen des brevets

(11) **EP 1 925 677 A2**

(12) **EUROPEAN PATENT APPLICATION**

(43) Date of publication:
**28.05.2008 Bulletin 2008/22**

(51) Int Cl.:
***C12Q 1/68*** *(2006.01)*

(21) Application number: **07119607.5**

(22) Date of filing: **03.07.2003**

(84) Designated Contracting States:
**AT BE BG CH CY CZ DE DK EE ES FI FR GB GR HU IE IT LI LU MC NL PT RO SE SI SK TR**

(30) Priority: **04.07.2002 GB 0215509**

(62) Document number(s) of the earlier application(s) in accordance with Art. 76 EPC:
**03762612.4 / 1 521 847**

(71) Applicants:
- **Novartis AG**
**4056 Basel (CH)**
Designated Contracting States:
**BE BG CH CY CZ DE DK EE ES FI FR GB GR HU IE IT LI LU MC NL PT RO SE SI SK TR**
- **Novartis Pharma GmbH**
**1235 Vienna (AT)**
Designated Contracting States:
**AT**

(72) Inventors:
- **Chibout, Salah-Dine**
**68720 Tagolsheim (FR)**

- **Grenet, Olivier**
**68730 Blotzheim (FR)**
- **Imbert, Georges**
**68220 Buschwiller (FR)**
- **Kehren, Jeanne**
**68490 Bantzenheim (FR)**
- **Stadtler, Frank**
**79591 Eimeldingen (DE)**
- **Wolfgang, Curt Douglas**
**Germantown, MD 20874 (US)**

(74) Representative: **Bourgarel, Denis Jacques Marie**
**Novartis AG**
**Corporate Intellectual Property**
**4002 Basel (CH)**

Remarks:
This application was filed on 30-10-2007 as a divisional application to the application mentioned under INID code 62.

(54) **Marker genes for determining renal toxicity**

(57) Methods are disclosed for fast and accurate readout of kidney toxicity before it occurs and before it is demonstrated by histopathology examination. Ultimately this approach shall allow earlier compound selection. The twelve genes identified, namely Calbindin-D28k, KIM-1, OPN, EGF, Clusterin, VEGF, OAT-K1, Aldolase A, Aldolase B, Podocin, Alpha-2u and C4, were grouped and ultimately can be assessed in the form of a kit using PCR, a high throughput technology, in order to characterize and rank new compounds according to their anticipated general kidney toxicity. Also disclosed are methods for identifying agents useful in the treatment of kidney disease, methods for monitoring the efficacy of a treatment for kidney disease and kidney-specific vectors including the sequences of the disclosed genes, and a method for identifying a candidate gene associated with a biological process including kidney function.

**Description**

**Field of the Invention**

The present invention relates to methods for the monitoring, prognosis, diagnostic and/or treatment of renal disorders, i.e. renal diseases, injuries or toxicities, to kits for diagnosing renal toxicity. In particular, the invention relates to the use of gene expression analysis to determine renal disorders and/or to help choosing or monitoring the efficacy of various treatments for renal disorders.

**Background**

The study of genetic and genomic factors at the DNA/RNA level involved in an individual's response to a foreign compound or drug permits the selection of safe agents (e.g., drugs) for prophylactic or therapeutic treatments. Agents or modulators which have a stimulatory or inhibitory effect on expression of a marker of the invention can be monitored in individuals to assess renal toxicity in the patient. Differences in metabolism of therapeutics can lead to severe toxicity or therapeutic failure by altering the relation between dose and blood concentration of the pharmacologically active drug. Such pharmacogenomics can further be used to determine appropriate dosages and therapeutic regimens. Accordingly, the level of expression of a marker of the invention in an individual can be determined to thereby select appropriate safe agent(s) for therapeutic or prophylactic treatment of the individual.

Pharmacogenetic deals with clinically significant variations in the efficacy or toxicity of drugs due to variations in drug disposition and action in individuals. See, e.g., Linder MW, Clin Chem 1997, Vol 43(2): 254-266. In general, two types of pharmacogenetic conditions can be differentiated. Genetic conditions transmitted as a single factor altering the way drugs act on the body are referred to as "altered drug action". Genetic conditions transmitted as single factors altering the way the body acts on drugs are referred to as "altered drug metabolism". These pharmacogenetic conditions can occur either as rare defects or as common polymorphisms.

The level of expression, or the level of function, of a marker in an individual can be determined to thereby select appropriate agent for therapeutic or prophylactic treatment of the individual. This knowledge, when applied to dosing or drug selection, can avoid adverse reactions or therapeutic failure, and thus enhance therapeutic or prophylactic efficiency when treating a subject with a modulator of expression of a marker.

**Calbindin D-28k** is a calcium-binding protein member of the large EF-hand family. It is present in all classes of vertebrates and in a wide range of tissues. Calbindin D-28k is postulated to function as a calcium transport molecule that facilitates the diffusion of calcium through the cell and serves as an intracellular calcium buffer maintaining the ionized calcium below toxic levels (Feher JJ, Am J Physiol 1983, Vol 44: C303-C307).

Several researchers have shown that in the kidney highest amounts of Calbindin D-28k are localized in the distal tubule, which correlates with the role of the distal tubule as the site of calcium absorption (Rhoten WB, et al., Anat Rec 1990, Vol 227: 145-151; Borke JL, et al., Am J Physiol (Renal Fluid Electrolyte Physiol) 1989, Vol 257: F842-F849 26.). Furthermore, it has been shown that the decreased expression of Calbindin D-28k with age may contribute to the age-related decrease in $Ca^{++}$ transport in intestine and kidney (Armbrecht HJ, et al., Endocrinology 1989, Vol 125: 2950-2956). It is reported in the literature that cyclosporine A-induced decrease in rat renal Calbindin-D 28k protein is a consequence of a decrease in its mRNA (Grenet O, et al., Biochem Pharmacol 1998, Vol 55(7): 1131-1133; Grenet O, et al., Biochem Pharmacol 2000; Vol 59(3): 267-272). This statement implies that the Calbindin-D28k mRNA levels can be monitored by PCR, which reflects protein levels (Steiner S, et al., Biochem Pharmacol 1996, Vol 51 (3): 253-258). These observations confirm the hypothesis that Calbindin D-28k has an important role in the re-absorption process taking place in the kidney.

**Kidney injury molecule-1** (KIM-1) is a type 1 membrane protein containing an extracellular, six-cysteine immunoglobulin domain. KIM-1 mRNA and protein are expressed at a low level in normal kidney but are increased dramatically in postischemic kidney. KIM-1 is localized to the regenerating, dedifferentiated proximal tubule epithelial cells, and absent in interstitial cells. KIM-1 is implicated in the restoration of the morphological integrity and function to post-ischemic kidneys (Ichimura T, et al., J Biol Chem 1998, Vol 273: 4135-4142).

**Osteopontin** (OPN), also known as secreted phosphoprotein 1 (SPP1), is a secreted, highly acidic and glycosylated phosphoprotein containing an arginine-glycine-aspartic acid (RGD) cell adhesion motif. OPN has originally been identified in osteoblasts and it was demonstrated to have the ability to bind hydroxyapatite and to play a major role in bone resorption, mineralization, and calcification (Reinholt FP, et al., Proc Natl Acad Sci U.S.A. 1990, Vol 87: 4473-4475). It was later shown to be localized in calcified atherosclerotic lesions and believed to be involved in the inhibition of calcium deposition of smooth muscle cells (Wada T, et al., Circ Res 1999, Vol 84: 166-178). Furthermore, high OPN expression was shown to be regulated by the vitamin $D_3$ active form (Noda M, et al., Proc Natl Acad Sci U. S. A. 1990, Vol 87: 9995-9999) and OPN has been found in tissues with high cell turnover. OPN up-regulation has been demonstrated in several models of renal injury, suggesting a possible role in tissue remodeling and repair (Persy VP, et al., Kidney Int 1999, Vol 56(2): 601-611). Osteopontin was also found to be a major component of urinary calcium oxalate stones (Kohri K, et al., Biochem Biophys Res Commun 1992, Vol 184: 859-864). Furthermore, OPN is highly expressed in distal tubular cells in rats prone to urinary stone formation (Kohri K, et al., J Biol Chem 1993, Vol 268: 15180-15184). These data lead to the hypothesis that Osteopontin is involved in urinary stone formation (Kohri K, et al., J Biol Chem 1993, Vol 268: 15180-15184).

**Epidermal growth factor** (EGF) is a small polypeptide belonging to a class of molecules that can mediate cell growth, differentiation, and acute phase responses. EGF mRNA is transcribed primarily in cells of the salivary gland and the kidney. In a variety of experimentally induced forms of acute renal failure, the mRNA and protein levels for kidney EGF fall markedly and remain low for a prolonged period (Price PM, et al., Am J Physiol 1995, Vol 268(4 Pt 2): F664-670). EGF is important epithelial mitogen. In addition, EGF receptor levels are known to play a central role in density dependent growth regulation of normal rat kidney fibroblasts (Lahaye DH, et al., FEBS Lett 1999, Vol 446(2-3): 256-260). EGF is involved in the endogenous tissue repair after acute renal injury. This growth factor accelerates with the recovery of renal function and the anatomical restoration of tubular integrity when given exogenously to laboratory animals with experimental acute renal failure (Wang S and Hirschberg R, Nephrol Dial Transplant 1997, Vol 12(8): 1560-1563). Furthermore, EGF is believed to play a major role in renal tubular regeneration after ischemic injury to the kidney (Di Paolo S, et al., Nephrol Dial Transplant 1997, Vol 12: 2687-2693) and in renal tissue repair after drug-induced nephrotoxicity (Morin NJ, et al., Am J Physiol 1992, Vol 263: F806-F811). EGF expression levels have been shown to be markedly reduced in renal transplantation patients suffering from chronic rejection or drug-induced nephrotoxicity (Di Paolo S, et al., Nephrol Dial Transplant 1997, Vol 12: 2687-2693). In addition, decreases in EGF expression in the kidney following cyclosporine A (CsA) treatment have been reported (Deng JT, et al., Transplant Proc 1994, Vol 26(5): 2842-2844; Yang CW, et al., Kindey Int 2001, Vol 60: 847-857).

**Clusterin**, also known as *Testosterone-repressed prostate message 2 (TRPM-2)* is aN ubiquitous, secreted glycoprotein induced in many organs, including the kidney, at times of tissue injury and/or remodeling, and it has been found in the tubular lumen of epithelial ducts (Jenne DE and Tschopp J, Trends Biochem Sci 1992, Vol 14: 154-159). It is believed to be involved in sperm maturation, lipid transport by forming high-density lipoprotein complexes with apolipoprotein A-I, membrane remodeling, inhibition of the complement cascade, neurodegeneration and apoptosis (Han B.H., et al., Nat Med 2001, Vol 7: 338-343; Wong P, et al., J Biol Chem 1993, Vol 268, 5021-5031; Wong P, et al., Eur J Biochem 1994, Vol 331: 917-925). Clusterin is a soluble complement regulatory protein that binds to C5b-7 and inhibits generation of membrane attack complex, C5b-9. Glomerular deposition of Clusterin has been observed in human and experimental membranous nephropathy in association with C5b-9 and immune deposits (Yamada K, et al., Kidney Int 2001, Vol 59(1): 137-146). It is speculated that Clusterin preserves cell interactions that are otherwise perturbed by renal insults (Silkensen JR, et al., J Am Soc Nephrol 1997, Vol 8(2): 302-305). Furthermore, it is reported that CsA increases Clusterin mRNA levels in the rat kidney (Darby IA, et al., Exp Nephrol 1995 Vol 3(4): 234-239).

**Alpha-2u globulin related-protein** (Alpha-2u), also known as Lipocalin 2 (LCN2) or Neutrophil Gelatinase-Associated Lipocalin (NGAL) in humans, is stored in granules of neutrophils and is associated with neutrophil gelatinase (Kjeldsen L, et al., J Biol Chem 1993, Vol 268: 10425-10432). It binds to small lipophilic substances and is believed to have a role in inflammation and embryogenesis (Bundgaard JR, et al., Biochem Biophys Res Commun 1994, Vol 202: 1468-1475; Cowland JB and Borregaard N, Genomics 1997, Vol 45: 17-23; Zerega B, et al., Eur J Cell Biol 2000, Vol 79, 165-172).

**Complement component 4** (C4) is constitutively expressed by renal tubular epithelial cells and is involved in modulating interstitial inflammation (Welch TR, et al., Clin Immunol 2001, Vol 101, 366-370). Decreases in its expression have been associated with increased renal disease activity in patients with systemic lupus erythematosus (Ho A, et al., Arthritis Rheum 2001, Vol 44: 2350-2357).

**Vascular Endothelial Growth Factor** (VEGF) is known to promote angiogenesis, increase vascular permeability, serve as a chemotactic for monocytes, and has a role in diabetes, wound healing, inflammatory responses, and tissue remodeling (Benjamin LE, Am J Pathol 2001, Vol 158: 1181-1184).

**Kidney-specific Organic Anion Transporter-K1** (OAT-K1), also known as solute carrier family 21 member a4 (SLC21A4), has homology to human solute carrier family 21 member a3 (SLC21A3). OAT-K1 is expressed in the basolateral membrane of kidney tubules and is involved in the renal clearance of drugs from the blood (Saito H, et al., J Biol Chem 1996, Vol 271: 20719-20725).

**Aldolase A** catalyzes the conversion of fructose-1,6-bisphosphate to glyceraldehyde 3-phosphate and dihydroxyacetone phosphate. It is found in the developing embryo and adult muscle and is repressed in adult liver, kidney and intestine. Aldolase A deficiency has been associated with myopathy and hemolytic anemia (Kishi H, et al., Proc Natl Acad Sci U.S.A. 1987, Vol 84: 8623-8627; Kreuder J, et al., N Engl J Med 1996, Vol 334: 1100-1104).

**Aldolase B** has similar functions to Aldolase A, and both isozymes are encoded by different genes. However, unlike Aldolase A, Aldolase B is expressed in adult liver, kidney, and intestine. A deficiency in Aldolase B has been linked to renal tubular acidosis and hereditary fructose intolerance (Cross NC, et al., Cell 1988, Vol 53: 881-885; Kranhold JF, et al., Science 1969, Vol 165: 402-403; Mass RE, et al., Am J Med Sci 1966, Vol 251: 516-523), indicating that Aldolase B may have a role in nephrotoxicity.

**Podocin**, also known as PDCN, SRN1, nephrosis 2, idiopathic, is a protein expressed in renal podocytes and plays a role in the regulation of glomerular permeability, acting probably as a linker between the plasma membrane and the cytoskeleton. It is almost exclusively expressed in the podocytes of fetal and mature kidney glomeruli. Mutation of podocyte proteins, e.g. podocin, result in congenital focal segmental glomerulosclerosis (Komatsuda A, et al., Ren Fail. 2003, Vol 25(1): 87-93) and is mainly implicated in steroid-resistant nephrotic syndrome.

Although some of the above markers are speculated to be associated with nephropathies, these markers have not been actually used alone or in combination as diagnostics, for selection of dosing or for selection of drug or for determining renal disorders, and their levels of expression have never been correlated to various renal disorder status.

In the field of renal nephropathies, there is however a need to allow election of appropriate agent for therapeutic or prophylactic treatment, prediction of an individual's drug responsiveness phenotype, and selection of dosing or drug in order to avoid adverse reactions or therapeutic failure.

Cyclosporine A (CsA; Neoral®) has been one of the hallmark immunosuppressants used for organ transplantations during the past 15 years for the prevention of graft rejection. However, CsA has been shown to induce nephrotoxicity that leads to chronic allograft nephropathy in renal transplantation patients. It is believed that CsA-induced nephrotoxicity is caused by a combination of the following events: increased concentrations of renin in the kidney (Masson J, et al., Kidney Int Suppl 1991, Vol 32, S28-S32), expression of TGF beta in the distal convoluted tubular epithelium (Langham RG, et al., Transplantation 2001, Vol 72: 1826-1829), increased intracellular calcium in vascular smooth muscle cells (Masson J, et al., Kidney Int Suppl 1991, Vol 32, S28-S32), increased prostacyclin release (Oriji GK, Prostaglandins Leukot Essent Fatty Acids 1999, Vol 61, 119-123), and increased thromboxane production in the kidney (Gonzalez-Correa JA, et al. Thromb Res 1996, Vol 81: 367-381).

## Summary of the Invention

The present invention relates to a method for determining renal toxicity in an individual comprising the steps of (a) obtaining a body sample from an individual, (b) determining from the body sample the level of gene expression corresponding to one or more genes selected among Calbindin-D28k, KIM-1, OPN, EGF, Clusterin, Alpha-2u, C4, VEGF, OAT-K1, Aldolase A, Aldolase B and Podocin, to obtain a first set of value, and (c) comparing the first set of value with a second set of value corresponding to the level of gene expression assessed for the same gene(s) and under identical condition as for step b) in a body sample of an individual not subject to renal toxicity, wherein the first value lower than the second value for Calbindin-D28k, EGF, VEGF, OAT-K1, Aldolase A, Aldolase B and/or Podocin, gene expression is an indication that the individual of step a) is having, developing or sensitive to renal toxicity, and/or wherein the first value greater than the second value for KIM-1, OPN, Clusterin, Alpha-2u and/or C4 gene expression is an indication that the individual is having, developing or sensitive to renal toxicity.

According to another aspect of the invention, the individual is under treatment with a cytotoxic agent such as cyclosporine, cisplatin, tacrolimus, aminoglycosides, sulfonamides or trimethadione.

The present invention further covers a test for use in determining whether a renal toxicity in an individual responds to therapy comprising the steps of, performing steps a), b) and c) of the invention for a body sample obtained from an individual treated against renal toxicity with a pharmaceutically acceptable agent and determining the responsiveness of the individual to drug therapy.

The present invention further covers another test for use in determining whether a kidney toxicity in an individual responds to therapy treatment comprising the steps of, performing steps a), b) and c) of the invention for a body sample obtained from an individual treated against renal toxicity with a pharmaceutically acceptable agent and determining the responsiveness of the individual to drug therapy.

In another aspect, the invention covers a method for treating renal toxicity in an individual comprising the step of administering to said individual a therapeutically effective amount of a modulating compound that modulates in the kidney the synthesis, expression or activity of one or more of the genes or gene expression products of the group of genes Calbindin-D28k, KIM-1, OPN, EGF, Clusterin, VEGF, OAT-K1, Aldolase A, Aldolase B, Podocin, Alpha-2u and/or C4, so that at least one symptom of renal toxicity is ameliorated.

According to another aspect of the invention, the individual is under treatment with a cytotoxic agent such as cyclosporine, cisplatin, tacrolimus, aminoglycosides, sulfonamides or trimethadione.

In a further aspect, the invention covers a method for identifying candidate agents for use in the treatment of renal toxicity comprising the steps of (a) contacting a sample of a kidney tissue subject to toxicity with a candidate agent, (b) determining from the kidney tissue the level of gene expression corresponding to one or more genes selected among Calbindin-D28k, KIM-1, OPN, EGF, Clusterin, VEGF, OAT-K1, Aldolase A, Aldolase B, Podocin, Alpha-2u and C4, to obtain a first set of value, and (c) comparing the first set of value with a second set of value corresponding to the level of gene expression assessed for the same gene(s) and under identical condition as for step b) in a kidney tissue subject to toxicity not induced by the candidate agent, wherein a first value substantially greater than the second value for Calbindin-D28k, EGF, VEGF, OAT-K1, Aldolase A, Aldolase B and/or Podocin gene expression is an indication that the candidate agent is ameliorating renal toxicity symptoms, and/or wherein a first value substantially lower than the second value for KIM-1, OPN, Clusterin, Alpha-2u and/or C4 gene expression is an indication that the candidate agent is ameliorating renal toxicity symptoms.

In a further aspect, the invention covers a method for identifying candidate agents that do not provoke or induce renal toxicity comprising the steps of (a) contacting a sample of a kidney tissue not subject to toxicity with a candidate agent, (b) determining from the kidney tissue the level of gene expression corresponding to one or more genes selected among Calbindin-D28k, KIM-1, OPN, EGF, Clusterin, VEGF, OAT-K1, Aldolase A, Aldolase B, Podocin, Alpha-2u and C4, to obtain a first set of value, and (c) comparing the first set of value(s) with a second set of value corresponding to the level of gene expression assessed for the same gene(s) and under identical condition as for step b) in a kidney tissue not subject to toxicity, wherein a first value equal or higher than the second value for Calbindin-D28k, EGF, VEGF, OAT-K1, Aldolase A, Aldolase B and/or Podocin gene expression is an indication that the candidate agent does not provoke or induce renal toxicity, and/or wherein a first value equal or lower than the second value for KIM-1, OPN, Clusterin, Alpha-2u and/or C4 gene expression is an indication that the candidate agent does not provoke or induce renal toxicity.

In a further aspect, the invention covers a method for comparing renal cytotoxic potentials of two drug candidates comprising the steps of (a) contacting a sample of a kidney tissue not subject to toxicity with a first drug candidate, and determining from the kidney tissue the level of gene expression corresponding to one or more genes selected among Calbindin-D28k, KIM-1, OPN, EGF, Clusterin, VEGF, OAT-K1, Aldolase A, Aldolase B, Podocin, Alpha-2u and C4, to obtain a first set of value, (b) contacting a sample of a kidney tissue not subject to toxicity with a second drug candidate, and determining from the kidney tissue level(s) of gene expression(s) corresponding to one or more genes selected among Calbindin-D28k, KIM-1, OPN, EGF, Clusterin, VEGF, OAT-K1, Aldolase A, Aldolase B, Podocin, Alpha-2u and C4, to obtain a second set of value, and (c) comparing the first set of value to the second set of value, wherein if the first value is substantially greater than the second value for Calbindin-D28k, EGF, VEGF, OAT-K1, Aldolase A, Aldolase B and/or Podocin gene expression this is an indication that the second drug candidate is less cytotoxic to the kidney than the first drug candidate, and/or wherein if the first value is substantially lower than the second value for KIM-1, OPN, Clusterin, Alpha-2u and/or C4 gene expression this is an indication that the second drug candidate is less cytotoxic to the kidney than the first drug candidate.

In a another aspect, the invention provides the use of a polymorphism in a gene for the diagnostic of renal toxicity, wherein the gene is chosen from Calbindin-D28k, KIM-1, OPN, EGF, Clusterin, VEGF, OAT-K1, Aldolase A, Aldolase B, Podocin, Alpha-2u and C4.

In a further aspect, the invention covers a kit for diagnosing renal toxicity in an individual comprising a means for determining the level of gene expression corresponding to one or more marker genes selected among Calbindin-D28k, KIM-1, OPN, EGF, Clusterin, VEGF, OAT-K1, Aldolase A, Aldolase B, Podocin, Alpha-2u and C4.

In other aspects of the invention, the individual is under treatment with a cytotoxic agent.

A last aspect of the invention covers a method for identifying a candidate gene associated with a biological process including kidney function, renal toxicity, and/or kidney disorders comprising the steps a) using a gene expression level of at least one marker selected among Calbindin-D28k, KIM-1, OPN, EGF, Clusterin, VEGF, OAT-K1, Aldolase A, Aldolase B, Podocin, Alpha-2u and C4 as input for an algorithm for obtaining at least one numerical value I; and b) comparing the at least one numerical value I obtained in a) with a numerical value II obtained for the candidate gene.

### Brief Description of the Drawings

Figure 1: Represents the evolution of the expression changes of gene markers linked to renal pathology status. The pathology scoring is defined as follows :1 = minimal, very few; 2 = slight, few; 3 = moderate, moderate number; 4 = marked, many; 5 = severe, extensive number.

Figure 2: Represents the occurrence of renal gene expression changes versus classical biochemical endpoint (creatinine levels). The pathology scoring is defined as follows :1 = minimal, very few; 2 = slight, few; 3 = moderate, moderate number; 4 = marked, many; 5 = severe, extensive number.

Figure 3: Represents relative fold expression-changes of marker genes in kidney of rat treated with two test compounds (TC1 and TC2) and Cyclosporine A (CsA). A.U.: Arbitrary units.

### Detailed Description of the Invention

As used herein the expression "renal toxicity" or "renal injury" or similarly "kidney disorder" shall all mean a renal or kidney failure or dysfunction either sudden (acute) or slowly declining over time (chronic), that may be triggered by a number of disease or disorder processes, including (but not limited to) for acute renal toxicity: sepsis (infection), shock, trauma, kidney stones, kidney infection, drug toxicity, poisons or toxins, or after injection with an iodinated contrast dye (adverse effect); and for chronic renal toxicity: long-standing hypertension, diabetes, congestive heart failure, lupus, or sickle cell anemia. Both forms of renal failure result in a life-threatening metabolic derangement.

The expression "body samples" shall include but is not limited to biopsies, preferably of the kidney, and body fluids such as blood, plasma, serum, lymph, cerebrospinal fluid, cystic fluid, ascites, urine, stool and bile, for instance. One advantage of the present invention is that one marker can be particularly well monitored in body fluids, such as plasma. For instance, clusterin's level of expression can be particularly well determined in plasma.

As used herein the term "Individual" shall mean a human person, an animal or a population or pool of individuals.

As used herein, the term "candidate agent" or "drug candidate" can be natural or synthetic molecules such as proteins or fragments thereof, antibodies, small molecule inhibitors or agonists, nucleic acid molecules, e.g., antisense nucleotides, ribozymes, double-stranded RNAs, organic and inorganic compounds and the like.

mRNA expression levels that are expressed in absolute values represent the number of molecules for a given gene calculated according to a standard curve. To perform quantitative measurements serial dilutions of a cDNA (standard) are included in each experiment in order to construct a standard curve necessary for the accurate mRNA quantitation. The absolute values (number of molecules) are given after extrapolation from the standard curve.

As used herein each marker referred to as "Calbindin-D28k", "KIM-1", "OPN", "EGF", "Clusterin", "VEGF", "OAT-K1", "Aldolase A", "Aldolase B", "Podocin", "Alpha-2u" or "C4" encompass the gene or gene product (including mRNA and protein) that are substantially similar to the markers identified below in Table 1.

In its broadest sense, the term "substantially similar", when used herein with respect to a nucleotide sequence, means a nucleotide sequence corresponding to a reference nucleotide sequence, wherein the corresponding sequence encodes a polypeptide having substantially the same structure and function as the polypeptide encoded by the reference nucleotide sequence, e.g. where only changes in amino acids not affecting the polypeptide function occur. Desirably the substantially similar nucleotide sequence encodes the polypeptide encoded by the reference nucleotide sequence. The percentage of identity between the substantially similar nucleotide sequence and the reference nucleotide sequence desirably is at least 80%, more desirably at least 85%, preferably at least 90%, more preferably at least 95%, still more preferably at least 99%. Sequence comparisons are carried out using a Smith-Waterman sequence alignment algorithm (see e.g. Waterman, M.S. Introduction to Computational Biology: Maps, sequences and genomes. Chapman & Hall. London: 1995. ISBN 0-412-99391-0). The localS program, version 1.16, is used with following parameters: match: 1, mismatch penalty: 0.33, open-gap penalty: 2, extended-gap penalty: 2.

A nucleotide sequence "substantially similar" to reference nucleotide sequence can also hybridize to the reference nucleotide sequence in 7% sodium dodecyl sulfate (SDS), 0.5 M $NaPO_4$, 1 mM EDTA at 50˚C with washing in 2X SSC, 0.1% SDS at 50˚C, more desirably in 7% sodium dodecyl sulfate (SDS), 0.5 M $NaPO_4$, 1 mM EDTA at 50˚C with washing in 1X SSC, 0.1 % SDS at 50˚C, more desirably still in 7% sodium dodecyl sulfate (SDS), 0.5 M $NaPO_4$, 1 mM EDTA at 50˚C with washing in 0.5X SSC, 0.1% SDS at 50˚C, preferably in 7% sodium dodecyl sulfate (SDS), 0.5 M $NaPO_4$, 1 mM EDTA at 50˚C with washing in 0.1 X SSC, 0.1% SDS at 50˚C, more preferably in 7% sodium dodecyl sulfate (SDS), 0.5 M $NaPO_4$, 1 mM EDTA at 50˚C with washing in 0.1X SSC, 0.1 % SDS at 65˚C, yet still encodes a functionally equivalent gene product.

The present invention provides a plurality of markers (Calbindin-D28k, KIM-1, OPN, EGF, Clusterin, VEGF, OAT-K1, Aldolase A, Aldolase B, Podocin, Alpha-2u and C4) that together or alone, are or can be used as markers of renal toxicity. In particularly useful embodiments, a plurality of these markers can be selected and their mRNA expression monitored simultaneously to provide expression profiles for use in various aspects.

In a preferred embodiment of the present methods, at least 2 or 3, or at least 5 or 7, or at least 9, 10, 11 or 12 markers selected among Calbindin-D28k, KIM-1, OPN, EGF, Clusterin, VEGF, OAT-K1, Aldolase A, Aldolase B, Podocin, Alpha-2u and C4 can be used for determination of their gene expression profiles.

As each marker can be linked to different renal pathological findings, it is possible to identify gene expression profiles of such markers that are particularly linked to a renal pathology. For instance, the Calbindin-D28k mRNA level is used as an early marker for calcium disturbance predictor for mineralization. The KIM-1 mRNA level is a marker for general kidney insult. The OPN mRNA level is an early marker for macrophage infiltration often associated with kidney toxicity and a marker for tissue remodeling upon renal injury. The EGF mRNA level is an early marker for general kidney toxicity. The Clusterin mRNA level is an early marker for immune-mediated kidney toxicity.

In a further preferred embodiment of the present methods, mRNA expression is assessed in the body samples or kidney tissues by techniques selected from the group consisting of Northern blot analysis, reverse transcription PCR, real time quantitative PCR, NASBA, TMA, or any other available amplification technology.

In another preferred embodiment of the present methods, the level of gene expression can alternatively be assessed by detecting the presence of a protein corresponding to the gene expression product.

It must be noted that mRNA expression levels expressed in absolute values in the present invention (see below) for Calbindin-D28k, KIM-1, OPN, EGF, Clusterin, VEGF, OAT-K1, Aldolase A, Aldolase B, Podocin, Alpha-2u and C4 are generally found in most population's type or species. These values may however possibly vary for each population's type or species. It may therefore be necessary to determine again for each marker the standard gene expression level for a targeted population's type or species which is not subject to renal toxicity, above or under which, as appropriate, renal toxicity symptoms can be found.

In a first particular aspect of the invention, a method is provided for determining renal toxicity in an individual, the steps comprise (a) obtaining a body sample from an individual; (b) determining from the body sample the level of gene expression corresponding to one or more genes selected among Calbindin-D28k, KIM-1, OPN, EGF and Clusterin, to obtain a first set of value; (c) and comparing the first set of value with a second set of value corresponding to the level of gene expression, assessed for the same gene(s) and under identical condition as for step b) in a body sample of an individual not subject to renal toxicity, wherein the first value lower than the second value for Calbindin-D28K and/or EGF gene expression is an indication that the individual of step a) is having, developing or sensitive to renal toxicity, and/or wherein the first value greater than the second value for KIM-1, Osteopontin and/or Clusterin gene expression is an indication that the individual of step a) is having, developing or sensitive to renal toxicity.

In another aspect of the invention a method is provided for determining renal toxicity in an individual, the steps comprise (a) obtaining a body sample from an individual; (b) determining from the body sample the level of gene expression corresponding to one or more genes selected among Alpha-2u globulin related-protein (Alpha-2u), Complement component 4 (C4), Vascular Endothelial Growth Factor (VEGF), Kidney-specific Organic Anion Transporter-K1 (OAT-K1), Aldolase A, Aldolase B and Podocin, to obtain a first set of value; and (c) comparing the first set of value with a second set of value corresponding to the level of gene expression assessed for the same gene(s) and under identical condition as for step b) in a body sample of an individual not subject to renal toxicity, wherein the first value lower than the second value for VEGF, OAT-K1, Aldolase A, Aldolase B and/or Podocin gene expression is an indication that the individual of step a) is having, developing or sensitive to renal toxicity, and/or wherein the first value greater than the second value for Alpha-2u and/or C4 gene expression is an indication that the individual is having, developing or sensitive to renal toxicity.

A further aspect of the invention provides for a method for determining renal toxicity in an individual under treatment with a cytotoxic agent comprising the steps (a) obtaining a body sample from said individual; (b) determining from the body sample the level of gene expression corresponding to one or more genes selected among Calbindin-D28k, KIM-1, OPN, EGF, Clusterin Alpha-2u, C4, VEGF, OAT-K1, Aldolase A, Aldolase B and Podocin, to obtain a first set of value; and (c) comparing the first set of value with a second set of value corresponding to the level of gene expression assessed for the same gene(s) and under identical condition as for step b) in a body sample of an individual not subject to renal toxicity, wherein the first value lower than the second value for Calbindin-D28K, EGF, VEGF, OAT-K1, Aldolase A, Aldolase B and/or Podocin gene expression is an indication that the individual of step a) is having, developing or sensitive to renal toxicity, and/or wherein the first value greater than the second value for KIM-1, OPN, Clusterin, Alpha-2u and/or C4 gene expression is an indication that the individual is having, developing or sensitive to renal toxicity. The cytotoxic agent may be any molecule having a known toxicity towards kidney, and may advantageously be selected from many examples that include: cyclosporine, cisplatin, aminoglycosides, sulfonamides, tacrolimus, trimethadione, etc. The cyclosporine may be an immunosuppressive cyclosporine such as cyclosporine A or ISAtx247, as e.g. described in WO99/18120 and WO 03/033527.

The mRNA expression level as determined in absolute value may be below 1.0E+06 for Calbindin-D28k, EGF, VEGF, OAT-K1, Aldolase A, Aldolase B and/or Podocin, and it may be above 1.0E+06 for KIM-1, Osteopontin, Clusterin, Alpha-2u and/or C4. The expression level may be below 1.0E+07 or below 1.0E+08 for Calbindin-D28k, EGF, VEGF, OAT-K1, Aldolase A, Aldolase B and/or Podocin, and/or above 1.0E+07 or above 1.0E+08 for KIM-1, Osteopontin, Clusterin, Alpha-2u and/or C4. The values may also for some marker genes and depending on population's type or species the mRNA expression level be above or below 1.0E+09.

In a preferred embodiment of such method, mRNA expression levels in the body sample of the individual of step a), of Calbindin-D28k below 5.30E+08, of KIM-1 above 1.50E+07, of EGF below 2.80E+08, of Osteopontin above 1.40E+08, of Clusterin above 1.90E+09, and/or Podocin below 3.00E+06, indicates that such individual is having, developing or sensitive to renal toxicity, wherein mRNA expression is determined in absolute value. These values may however possibly vary for each population's type or species. It may therefore be necessary to determine again for each marker the standard gene expression level for a targeted population's type or specie which is not subject to renal toxicity, above or under which, as appropriate, renal toxicity symptoms can be found.

The mRNA expression levels may also be measured in relative values for Calbindin-D28k, KIM-1, OPN, EGF, Clusterin, VEGF, OAT-K1, Aldolase A, Aldolase B, Podocin, Alpha-2u and C4. These values may however may vary for each population's type or species. It may therefore be necessary to determine again for each marker the standard gene expression level for a targeted population's type or species which is not subject to renal toxicity. It may be that an individual is having, developing or sensitive to renal toxicity when the mRNA expression value for EGF, VEGF, OAT-K1, Aldolase A, Aldolase B and/or Podocin is at least 2 fold lower, and/or at least 2 fold greater for KIM-1, OPN, Clusterin, Alpha-2u and/or C4. Expression may be 5 fold lower for EGF, VEGF, OAT-K1, Aldolase A, Aldolase B and/or Podocin and/or 5 fold greater for KIM-1, OPN, Clusterin, Alpha-2u and/or C4, expression may also be 10, 20, 30, 40, 50, or 60 fold lower or greater respectively, when compared to the expression in a body sample of an individual not subject to renal toxicity.

In a preferred embodiment of such method the first value is at least 4 fold lower for EGF, at least 2 fold lower for VEGF, at least 2 fold lower for OAT-K1, at least 20 fold lower for Aldolase A, and/or for Aldolase B at least 2 fold lower than the second value, and/or the first value is at least 20 fold greater for KIM-1, at least 3 fold greater for OPN, at least 7 fold greater for Clusterin, at least 50 fold greater for Alpha-2u and/or for C4 at least 3 fold greater than the second value indicating that such individual is having, developing or sensitive to renal toxicity.

In another preferred embodiment of the invention, the first value is at least 4.5 fold lower for EGF, at least 2.6 fold lower for VEGF, at least 2.3 fold lower for OAT-K1, at least 26 fold lower for Aldolase A, and/or for Aldolase B at least 2.1 fold lower than the second value, and/or the first value is at least 26 fold greater for KIM-1, at least 3.9 fold greater for OPN, at least 7.6 fold greater for Clusterin, at least 60 fold greater for Alpha-2u and/or for C4 at least 3.3 fold greater than the second value indicating that such individual is having, developing or sensitive to renal toxicity.

In another particular aspect of the invention, the expression profiles of one or a plurality of these markers could provide valuable molecular tools for examining the molecular basis of drug responsiveness in renal toxicity and for evaluating the efficacy of drugs for treating renal toxicity or their side effects on the kidney. Changes in the expression profile from a baseline profile while the cells are exposed to various modifying conditions, such as contact with a drug or other active molecules can be used as an indication of such effects.

Therefore, the invention provides a test for use in determining whether a renal toxicity in a patient will respond to therapy comprising the steps of, performing steps a), b) and c) of the method above for body samples obtained respectively from an individual treated against renal toxicity with a pharmaceutically acceptable agent and an individual not subject to renal toxicity, and determining the responsiveness to drug therapy.

Monitoring the influence of agents (e.g., drug compounds) on the level of expression of a marker of the invention can be advantageously applied in clinical trials. For example, the effectiveness of an agent to affect marker expression can be monitored in clinical trials of subjects receiving treatment for renal disease or toxicity. In a preferred embodiment, the present invention provides a method for monitoring the effectiveness of treatment of a subject with an agent (e.g., an agonist, antagonist, peptidomimetic, protein, peptide, nucleic acid, small molecule, or other drug candidate) comprising the steps of: (i) obtaining a pre-administration sample from a subject prior to administration of the agent; (ii) detecting the level of expression of one or more selected markers of the invention in the pre-administration sample; (iii) obtaining one or more post-administration samples from the subject; (iv) detecting the level of expression of the marker(s) in the post-administration samples; (v) comparing the level of expression of the marker(s) in the pre-administration sample with the level of expression of the marker(s) in the post-administration sample or samples; and (vi) altering the administration of the agent to the subject accordingly. For example, modified administration of the agent can be desirable to increase expression of the marker(s) to higher levels than detected, i.e., to increase the effectiveness of the agent. Alternatively, increased/decreased administration of the agent can be desirable to increase/decrease the effectiveness of the agent, respectively.

In another particular aspect of the present invention, a method is provided for both prophylactic and therapeutic methods of treating a subject having, or at risk of having, a kidney disorder or renal toxicity. Administration of a prophylactic agent can occur prior to the manifestation of symptoms characteristic of the kidney disorder, such that development of the kidney disorder is prevented or delayed in its progression. Examples of suitable therapeutic agents include, but are not limited to, antisense nucleotides, ribozymes, double-stranded RNAs, ligands, small molecules and antagonists as described more in detail below.

In a particular embodiment, the invention provides a method for treating or preventing renal toxicity in an individual comprising the step of administering to said individual a therapeutically effective amount of a modulating compound that modulates in the kidney the synthesis, expression or activity of one or more of the genes or gene expression products of the group of genes Calbindin-D28k, KIM-1, OPN, EGF and/or Clusterin, so that at least one symptom of renal toxicity is ameliorated.

In another aspect, the invention provides a method for treating renal toxicity in an individual comprising the step of administering to said individual a therapeutically effective amount of a modulating compound that modulates in the kidney the synthesis, expression or activity of one or more of the genes or gene expression products of the group of genes VEGF, OAT-K1, Aldolase A, Aldolase B, Podocin, Alpha-2u and/or C4, so that at least one symptom of renal toxicity is ameliorated.

According to a further aspect of the invention a method for treating renal toxicity in an individual under treatment with a cytotoxic agent is provided, comprising the step of administering to said individual a therapeutically effective amount of a modulating compound that modulates in the kidney the synthesis, expression or activity of one or more of the genes or gene expression products of the group of genes Calbindin-D28k, KIM-1, OPN, EGF, Clusterin, VEGF, OAT-K1, Aldolase A, Aldolase B, Podocin, Alpha-2u and/or C4, so that at least one symptom of renal toxicity is ameliorated. The cytotoxic agent is preferably selected among cyclosporine, cisplatin, tacrolimus, aminoglycosides, sulfonamides and trimethadione.

In a particular embodiment of the invention a gene mRNA expression in a body sample of an individual after treatment with the modulating compound, of Calbindin-D28k above to 5.30E+08, of KIM-1 below 1.50E+07, of EGF above 2.80E+08, of Osteopontin below 1.40E+08, of Clusterin below 1.90E+09 and/or of Podocin above 3.00E+06, indicates that at least one symptom of renal toxicity is ameliorated, wherein gene mRNA expression is determined in absolute value. These values may however possibly vary for each population's type or species.

In another particular embodiment, a repression of gene expression measured in a body sample of an individual after treatment with the modulating compound, of less than 4 fold for EGF, of less than 2 fold for VEGF, of less than 2 fold for OAT-K1, of less than 20 fold for Aldolase A, and/or for Aldolase B of less than 2 fold, and/or an induction of gene expression of less than 20 fold for KIM-1, of less than 3 fold for OPN, of less than 7 fold for Clusterin, of less than 50 fold for Alpha-2u and/or for C4 of less than 3 fold, indicates that at least one symptom of renal toxicity is ameliorated. These values may however possibly vary for each population's type or species.

In another particular aspect of the invention, by virtue of the differential expression of the markers, it is possible to utilize these markers to enhance the certainty of prediction of whether a particular drug treatment in a patient will not be toxic to the kidney. Therefore, the invention provides a method for identifying candidate agents for use in the treatment of renal toxicity comprising the steps of: a) contacting a sample of a kidney tissue subject to toxicity with a candidate agent; b) determining from the kidney tissue the level of gene expression corresponding to one or more genes selected among Calbindin-D28k, KIM-1, OPN, EGF and Clusterin, to obtain a first set of value; and c) comparing the first set of value with a second set of value corresponding to the level of gene expression, assessed for the same gene(s) and under identical condition as for step b) in a kidney tissue subject to toxicity not induced by the candidate agent, wherein a first value substantially equal or greater than the second value for Calbindin-D28K and/or EGF gene expression is an indication that the candidate agent is ameliorating renal toxicity symptoms, and/or wherein a first value substantially equal or lower than the second value for KIM-1, Osteopontin and/or Clusterin gene expression is an indication that the candidate agent is ameliorating renal toxicity symptoms.

In another particular aspect of the invention, a method is provided for identifying candidate agents for use in the treatment of renal toxicity comprising the steps of (a) contacting a sample of a kidney tissue subject to toxicity with a candidate agent; (b) determining from the kidney tissue the level of gene expression corresponding to one or more genes selected among VEGF, OAT-K1, Aldolase A, Aldolase B, Podocin, Alpha-2u and C4, to obtain a first set of value; and (c) comparing the first set of value with a second set of value corresponding to the level of gene expression assessed for the same gene(s) and under identical condition as for step b) in a kidney tissue subject to toxicity not induced by the candidate agent wherein a first value substantially greater than the second value for VEGF, OAT-K1, Aldolase A, Aldolase B and/or Podocin gene expression is an indication that the candidate agent is ameliorating renal toxicity symptoms, and/or wherein a first value substantially lower than the second value for Alpha-2u and/or C4 gene expression is an indication that the candidate agent is ameliorating renal toxicity symptoms.

In a preferred embodiment, mRNA gene expression in kidney tissue subject to toxicity, of Calbindin-D28k above 5.30E+08, of KIM-1 below 1.50E+07, of EGF above 2.80E+08, of Osteopontin below 1.40E+08, of Clusterin below 1.90E+09, and/or of Podocin above 3.00E+06, is an indication that the candidate agent is ameliorating renal toxicity, wherein mRNA gene expression is determined in absolute value. These values may however possibly vary for each population's type or specie.

In another preferred embodiment, a repression of gene expression of less than 4 fold for EGF, of less than 2 fold for VEGF, of less than 2 fold for OAT-K1, of less than 20 fold for Aldolase A, and/or for Aldolase B of less than 2 fold, and/or an induction of gene expression of less than 20 fold for KIM-1, of less than 3 fold for OPN, of less than 7 fold for Clusterin, of less than 50 fold for Alpha-2u and/or for C4 of less than 3 fold, is an indication that the candidate agent is ameliorating renal toxicity.

In another particular aspect of the present invention, a method is provided for identifying candidate agents that do not provoke or induce renal toxicity comprising the steps of: a) contacting a sample of a kidney tissue not subject to toxicity with a candidate agent; b) determining from the kidney tissue the level of gene expression corresponding to one or more genes selected among Calbindin-D28k, KIM-1, OPN, EGF and Clusterin, to obtain a first set of value; and c) comparing the first set of value with a second set of value corresponding to the level of gene expression assessed for the same gene(s) and under identical condition as for step b) in a kidney tissue not subject to toxicity, wherein a first value substantially equal or greater than the second value for Calbindin-D28K and/or EGF gene expression is an indication that the candidate agent does not provoke or induce renal toxicity, and/or wherein a first value substantially equal or lower than the second value for KIM-1, Osteopontin and/or Clusterin gene expression is an indication that the candidate agent does not provoke or induce renal toxicity.

In another particular aspect of the present invention, a method is provided for identifying candidate agents that do not provoke or induce renal toxicity comprising the steps of: a) contacting a sample of a kidney tissue not subject to toxicity with a candidate agent; b) determining from the kidney tissue the level of gene expression corresponding to one or more genes selected among VEGF, OAT-K1, Aldolase A, Aldolase B, Podocin, Alpha-2u and C4, to obtain a first set of value; and c) comparing the first set of value with a second set of value corresponding to the level of gene expression assessed for the same gene(s) and under identical condition as for step b) in a kidney tissue not subject to toxicity, wherein a first value equal or higher than the second value for VEGF, OAT-K1, Aldolase A, Aldolase B, and/or Podocin, gene expression is an indication that the candidate agent does not provoke or induce renal toxicity, and/or wherein a first value equal or lower than the second value for Alpha-2u and/or C4 gene expression is an indication that the candidate agent does not provoke or induce renal toxicity.

In a preferred embodiment, mRNA expression levels determined in the kidney tissue not subject to toxicity, of Calbindin-D28k above 5.30E+08, of KIM-1 below 1.50E+07, of EGF above 2.80E+08, of Osteopontin below 1.40E+08, of Clusterin below 1.90E+09, and/or of Podocin above 3.00E+06; is an indication that the candidate agent does not provoke or induce renal toxicity, wherein mRNA expression is determined in absolute value. These values may however possibly vary for each population's type or specie. The values may however possibly vary for each population's type or specie.

According to another preferred embodiment of the invention, a repression of gene expression of less than 4 fold for EGF, of less than 2 fold for VEGF, of less than 2 fold lower for OAT-K1, of less than 20 fold lower for Aldolase A, and/or for Aldolase B of less than 2 fold lower than the second value, and/or an induction of gene expression of less than 20 fold for KIM-1, of less than 3 fold for OPN, of less than 7 fold for Clusterin, of less than 50 fold for Alpha-2u and/or for C4 of less than 3 fold is an indication that the candidate agent does not provoke or induce renal toxicity. These values may also vary for each population's type or species.

In another particular aspect of the present invention, a method is provided for comparing renal cytotoxic potentials of two drug candidates comprising the steps of: a) contacting a sample of a kidney tissue not subject to toxicity with a first drug candidate, and determining from the kidney tissue level(s) of gene expression(s) corresponding to one or more genes selected among Calbindin-D28k, KIM-1, OPN, EGF and Clusterin, to obtain a first value; and b) contacting a sample of a kidney tissue not subject to toxicity with a second drug candidate, and determining from the kidney tissue level(s) of gene expression(s) corresponding to one or more genes selected among Calbindin-D28k, KIM-1, OPN, EGF and Clusterin, to obtain a second value; and c) comparing the first value with the second value, wherein if the first value is substantially lower than the second value for Calbindin-D28K and/or EGF gene expression(s) this is an indication that the second drug candidate is less cytotoxic to the kidney than the second drug candidate, and/or wherein if the first value is substantially higher than the second value for KIM-1, Osteopontin and/or Clusterin gene expression(s) this is an indication that the second drug candidate is less cytotoxic to the kidney than the second drug candidate.

In a further particular aspect of the present invention, a method is provided for comparing renal cytotoxic potentials of two drug candidates comprising the steps of: a) contacting a sample of a kidney tissue not subject to toxicity with a first drug candidate, and determining from the kidney tissue the level of gene expression corresponding to one or more genes selected among VEGF, OAT-K1, Aldolase A, Aldolase B, Podocin, Alpha-2u and C4, to obtain a first set of value; and b) contacting a sample of a kidney tissue not subject to toxicity with a second drug candidate, and determining from the kidney tissue level(s) of gene expression(s) corresponding to one or more genes selected among VEGF, OAT-K1, Aldolase A, Aldolase B, Podocin, Alpha-2u and C4, to obtain a second set of value; and c) comparing the first set of value to the second set of value, wherein if the first value is substantially lower than the second value for VEGF, OAT-K1, Aldolase A, Aldolase B, and/or Podocin gene expression this is an indication that the second drug candidate is less cytotoxic to the kidney than the second drug candidate, and/or wherein if the first value is substantially higher than the second value for Alpha-2u and/or C4 gene expression this is an indication that the second drug candidate is less cytotoxic to the kidney than the second drug candidate.

One particular advantage of the above methods, i.e., methods (i) for identifying candidate agents, (ii) for comparing renal cytotoxic potentials of two drug candidates and (iii) for identifying candidate agents that do not provoke or induce renal toxicity, is that they an be performed *in-vitro.* The kidney tissues that are used are preferably obtained from a cultured kidney tissue or cells that have been contacted with a cytotoxic agent. The kidney tissue can also be a kidney sample of an individual subject to renal toxicity, but this may limit broad *in-vitro* applications of such methods.

Cultured kidney tissue or cells may be advantageously based on an *in vivo* animal model that mimics human cellular and tissues disorders, preferably of the kidney. It may also be a single or collection of kidney cells such as the human kidney epithelial 293Tcells or a human embryonic kidney cell line, for instance. The cytotoxic agent may be any molecule having a known toxicity towards kidney, and may advantageously be selected from many examples that include: cyclosporine, cisplatin, aminoglycosides, sulfonamides, tacrolimus, trimethadione, etc. The kidney is particularly susceptible to the nephrotoxic action of drugs, because of its functional properties, including: a) the high volume of renal blood flow, which brings large amounts of toxin; b) the large area in contact with the drug, either in the glomerulus or the tubule epithelium, which enables toxin interaction or uptake; c) the kidney's ability to transfer active substances, which provides specific transfer mechanisms that mediate cellular uptake; d) drug breakdown, which may occur in renal tubules and lead to the formation of toxic metabolites from non-toxic parent substances; e) the kidney's concentrating mechanisms, which can increase urinary and interstitial concentrations of non-absorbed products; f) the high metabolic rate of tubule cells required for normal function, which is subject to perturbation.

The concentration of cyclosporine (e.g. Neoral®) can range from 10E-11 to 10E-5 M in the case of *in vitro* studies. These values may however possibly vary for each population's cell type or culture conditions.

A further particular aspect of the present invention provides a kit for diagnosing renal toxicity in an individual comprising a means for determining the level of gene expression corresponding to one or more marker genes selected among Calbindin-D28k, KIM-1, OPN, EGF, Clusterin, VEGF, OAT-K1, Aldolase A, Aldolase B, Podocin, Alpha-2u and C4.

A preferred embodiment provides a kit for diagnosing renal toxicity in an individual under treatment with a cytotoxic agent. Cyclosporine, cisplatin, tacrolimus, aminoglycosides, sulfonamides and/or trimethadione are preferably the cytotoxic agent.

In a particular embodiment of the present invention a kit is provided, wherein the level of gene expression of at least 2 or 3 genes selected among Calbindin-D28k, KIM-1, OPN, EGF, Clusterin, VEGF, OAT-K1, Aldolase A, Aldolase B, Podocin, Alpha-2u and C4 can be determined.

In a preferred embodiment, the means for determining the level of gene expression comprise oligonucleotides specific for a marker gene. Particularly preferred are methods selected from Northern blot analysis, reverse transcription PCR or real time quantitative PCR, branched DNA, nucleic acid sequence based amplification (NASBA), transcription-mediated amplification, ribonuclease protection assay, and microarrays.

Another particular embodiment provides a kit, wherein the means for determining the level of gene expression comprise at least one antibody specific for a protein encoded by the marker gene selected among Calbindin-D28k, KIM-1, OPN, EGF, Clusterin, VEGF, OAT-K1, Aldolase A, Aldolase B, Podocin, Alpha-2u and C4. The antibody is preferably selected among polyclonal antibodies, monoclonal antibodies, humanized or chimeric antibodies, and biologically functional antibody fragments sufficient for binding of the antibody fragment to the marker. Particularly preferred are immunoassay methods for determining the level of gene expression.

In another preferred embodiment of the invention a kit is provided which further comprises means for obtaining a body sample of the individual. A particularly preferred embodiment further comprises a container suitable for containing the means for determining the level of gene expression and the body sample of the individual. In another preferred embodiment the kit further comprises instructions for use and interpretation of the kit results.

Measurement methods

A particularly useful method for detecting the level of mRNA transcripts obtained from the markers (Calbindin-D28k, KIM-1, OPN, EGF, Clusterin, VEGF, OAT-K1, Aldolase A, Aldolase B, Podocin, Alpha-2u and C4) involves hybridization of labeled mRNA to an ordered array of oligonucleotides. Such a method allows the level of transcription of a plurality of these genes to be determined simultaneously to generate gene expression profiles or patterns. The gene expression profile derived from the sample obtained from the subject can, in another embodiment, be compared with the gene expression profile derived form the sample obtained from the disease-free subject, and thereby determine whether the subject has or is at risk of developing renal disease or toxicity.

The gene expressions of the markers (Calbindin-D28k, KIM-1, OPN, EGF, Clusterin, VEGF, OAT-K1, Aldolase A, Aldolase B, Podocin, Alpha-2u and C4) can be preferably assessed in the form of a kit using RT-PCR, a high throughput technology: The well-known technique RT-PCR reaction exploits the 5' nuclease activity of AmpliTaq Gold DNAPolymerase to cleave a TaqMan probe during PCR. The probe consists of an oligonucleotide (usually $\approx$20 mer) with a 5'-reporter dye and a 3'-quencher dye. The fluorescent reporter dye, such as FAM (6-carboxyfluorescein), is covalently linked to the 5' end of the oligonucleotide. The reporter is quenched by TAMRA (6-carboxy-N,N,N',N'-tetramethylrhodamine) attached via a linker arm that is located at the 3' end.

Oligonucleotide probes used for each marker (Calbindin-D28k, KIM-1, OPN, EGF, Clusterin, VEGF, OAT-K1, Aldolase A, Aldolase B, Podocin, Alpha-2u and C4) should derive from the nucleotide sequence of the gene of such marker, the selection of the appropriate oligonucleotide sequence being now a matter of standard routine technique for one skilled in the art. The following Table 1 gives various access codes of the Genbank database for marker sequences in humans, rat and/or mouse.

Table 1: Sequences of the marker genes

| Gene name | Calbindin-D28k (CALB1) | KIM-1 | Osteopontin (Uropontin, SPP-1) | Clusterin (TRPM-2; Apolipoprot. J) |
|---|---|---|---|---|
| Genbank # | **Rat:** M31178 | **Rat:** AF035963 | **Rat:** AB001382 M99252 | **Rat:** U02391 M64723 |
| Genbank # | **Human:** NM_004929 AC004612 AF049895 | **Human:** AL159977 AC073225.5 AC025449.6 | **Human:** AF052124 D14813 J04765 | **Human:** AF311103 J02908 L00974 |

(continued)

| Gene name | Calbindin-D28k (CALB1) | KIM-1 | Osteopontin (Uropontin, SPP-1) | Clusterin (TRPM-2; Apolipoprot. J) |
|---|---|---|---|---|
| | AF068862<br>AF070717<br>BC006478<br>M19878<br>M19879<br>X06661 | AF165926<br>AL449103 | M83248<br>U20758<br>X13694 | M25915<br>M63379<br>M64722<br>M74816<br>X14723 |
| Genbank # | **Mouse:**<br>AK002635<br>AK005081<br>AK005243<br>D26352<br>D26353<br>D26354<br>D26355<br>D26356<br>D26357<br>M21531<br>M23663 | **Mouse:**<br>A1662116 | **Mouse:**<br>J04806<br>M38399<br>S78177<br>X13986<br>X14882<br>X16151<br>X51834 | **Mouse:**<br>AF182509<br>D14077<br>L05670<br>L08235<br>S70244 |
| **Gene name** | **EGF** | **Alpha-2u (Lipocalin 2)** | **C4** | **VEGF** |
| Genbank # | **Rat:**<br>AF187818 | **Rat:**<br>NM_130741<br>AA946503 | **Rat:**<br>U42719<br>BI285347<br>AA800942<br>A1103841 | **Rat:**<br>AA850734<br>AF080594<br>M32167 |
| Genbank # | **Human:**<br>J02548<br>X04571 | **Human:**<br>NM_005564.1<br>BC033089<br>X83006<br>X83006.1<br>X99133.1<br>XM_209970 | **Human:**<br>NM_000592<br>NM_007293<br>K02403<br>AI983615<br>R37128 | **Human:**<br>AF024710<br>AF022375<br>AF091352<br>M27281<br>AF022375<br>AW024572<br>M27281<br>M32977 |
| Genbank # | **Mouse:**<br>J00380<br>U69534<br>V00741<br>X08047 | **Mouse:**<br>AK002932<br>AV230461<br>NT_039205<br>X14607X81627<br>XM_130171 | **Mouse:**<br>AV259769<br>X06454<br>AI661626<br>NM_009780<br>AV259769<br>M11729 | **Mouse:**<br>U43836 |
| **Gene name** | **OAT-K1** | **Aldolase A** | **Aldolase B** | **Podocin** |
| Genbank# | **Rat:**<br>D79981 | **Rat:**<br>U20643<br>NM_012495 | **Rat:**<br>X02284<br>M10149 | **Rat:**<br>NM_130828<br>AY039651 |

(continued)

| Gene name | EGF | Alpha-2u (Lipocalin 2) | C4 | VEGF |
|---|---|---|---|---|
| | | M12919<br>AA924326<br>AI102716 | X02291 | |
| Genbank # | **Human:**<br>NM_021094<br>AF085224<br>U21943<br>N62948 | **Human:**<br>NM_000034<br>AK026577<br>X05236<br>AI921586<br>X12447 | **Human:**<br>NM_000035<br>AK026411<br>X02747<br>AI469183<br>H91325 | **Human:**<br>NM_014625.1<br>AJ279246.1<br>AJ279254<br>AJ279254.1<br>BC029141 |
| Genbank # | | **Mouse:**<br>Y00516<br>AA717247 | | **Mouse:**<br>AJ302048<br>AW106985<br>AY050309 |

The protein expressions of the markers (Calbindin-D28k, KIM-1, OPN, EGF, Clusterin, VEGF, OAT-K1, Aldolase A, Aldolase B, Podocin, Alpha-2u and C4) that are secreted by both normal and disease cells can be also analyzed and are of value in the methods of this invention. Supernatants can be isolated and MWT-CO filters can be used to simplify the mixture of proteins. The proteins can then be digested with trypsin. The tryptic peptides may then be loaded onto a microcapillary HPLC column where they are separated, and eluted directly into an ion trap mass spectrometer, through a custom-made electrospray ionization source. Throughout the gradient, sequence data can be acquired through fragmentation of the four most intense ions (peptides) that elute off the column, while dynamically excluding those that have already been fragmented. In this way, the sequence data from multiple scans can be obtained, corresponding to approximately 50 to 200 different proteins in the sample. These data are searched against databases using correlation analysis tools, such as MS-Tag, to identify the protein expressions of the markers in the supernatants.

Expression of the protein encoded by the markers can also be detected by a probe which is detectably labeled, or which can be subsequently labeled. Generally, the probe is an antibody that recognizes the expressed protein.

As used herein, the term antibody includes, but is not limited to, polyclonal antibodies, monoclonal antibodies, humanized or chimeric antibodies, and biologically functional antibody fragments sufficient for binding of the antibody fragment to the protein.

The extent to which the known proteins are expressed in the sample is then determined by immunoassay methods that utilize the antibodies described above. Such immunoassay methods include, but are not limited to, dot blotting, western blotting, competitive and noncompetitive protein binding assays, enzyme-linked immunosorbant assays (ELISA), immunohistochemistry, fluorescence activated cell sorting (FACS), and others commonly used and widely described in scientific and patent literature, and many employed commercially.

Alternatively, marker proteins (Calbindin-D28k, KIM-1, OPN, EGF, Clusterin, VEGF, OAT-K1, Aldolase A, Aldolase B, Podocin, Alpha-2u and C4) can be separated by two-dimensional gel electrophoresis systems. Two-dimensional gel electrophoresis is well known in the art and typically involves iso-electric focusing along a first dimension followed by SDS-PAGE electrophoresis along a second dimension. The resulting electropherograms can be analyzed by numerous techniques, including mass spectrometric techniques, western blotting and immunoblot analysis using polyclonal and monoclonal antibodies, and internal and N-terminal micro-sequencing.

Drug screening methods

In addition to the drug screening methods described above, cell-free assays can also be used to identify compounds which are capable of interacting with proteins encoded by the markers (Calbindin-D28k, KIM-1, OPN, EGF, Clusterin, VEGF, OAT-K1, Aldolase A, Aldolase B, Podocin, Alpha-2u and C4), to alter the activity of the protein or its binding partner. Cell-free assays can also be used to identify compounds, which modulate the interaction between the encoded protein and its binding partner such as a target peptide.

In one embodiment, cell-free assays for identifying such compounds comprise a reaction mixture containing a marker protein (Calbindin-D28k, KIM-1, OPN, EGF, Clusterin, VEGF, OAT-K1, Aldolase A, Aldolase B, Podocin, Alpha-2u and C4) and a test compound or a library of test compounds in the presence or absence of the binding partner, e.g.,

a biologically inactive target peptide, or a small molecule. Interaction between molecules can also be assessed by using real-time BIA (Biomolecular Interaction Analysis, Pharmacia Biosensor (AB) which detects surface plasmon resonance, an optical phenomenon. Formation of a complex between the protein and its binding partner can be detected by using detectably labeled proteins such as radiolabeled, fluorescently labeled, or enzymatically labeled protein or its binding partner, by immunoassay or by chromatographic detection.

Transcript array

In a preferred embodiment the present invention makes use of "oligonucleotide arrays" (also called herein "microarrays"). Microarrays can be employed for analyzing the transcriptional state in a cell, and especially for measuring the transcriptional states of kidney cells.

In one embodiment, transcript arrays are produced by hybridizing detectably labeled polynucleotides representing the mRNA transcripts present in a cell (e.g., fluorescently labeled cDNA synthesized from total cell mRNA or labled cRNA.) to a microarray. A microarray in the present invention is a surface with an ordered array of binding (e.g., hybridization) sites for products of at least one of the marker genes (Calbindin-D28k, KIM-1, OPN, EGF, Clusterin, VEGF, OAT-K1, Aldolase A, Aldolase B, Podocin, Alpha-2u and C4). Microarrays can be made in a number of ways. However produced, microarrays share certain characteristics: The arrays are reproducible, allowing multiple copies of a given array to be produced and easily compared with each other. Preferably the microarrays are small, usually smaller than 5 cm.sup.2, and they are made from materials that are stable under binding (e.g. nucleic acid hybridization) conditions. A given binding site or unique set of binding sites in the microarray will specifically bind the product of a single gene in the cell. Although there may be more than one physical binding site (hereinafter "site") per specific mRNA, for the sake of clarity the discussion below will assume that there is a single site. In a specific embodiment, positionally addressable arrays containing affixed nucleic acids of known sequence at each location are used.

It will be appreciated that when cDNA complementary to the RNA of a cell is made and hybridized to a microarray under suitable hybridization conditions, the level of hybridization to the site in the array corresponding to any particular gene will reflect the prevalence in the cell of mRNA transcribed from that gene. For example, when detectably labeled (e.g., with a fluorophore) cDNA or cRNA complementary to the total cellular mRNA is hybridized to a microarray, the site on the array corresponding to a gene (i.e., capable of specifically binding the product of the gene) that is not transcribed in the cell will have little or no signal (e.g., fluorescent signal), and a gene for which the encoded mRNA is prevalent will have a relatively strong signal.

Databases

This invention also provides a process for preparing a database comprising gene expression profiles for at least one of the markers set forth in this invention (Table 1). For example, the gene expression profiles for each marker can be stored in a digital storage medium such that a data processing system for standardized representation of the markers profiles, alone or in combination, that identify a particular renal disease or toxicity cell is compiled.

Alternative computer systems and methods for implementing the analytic methods of this invention will be apparent to one of skill in the art and are intended to be comprehended within the accompanying claims. In particular, the accompanying claims are intended to include the alternative program structures for implementing the methods of this invention that will be readily apparent to one of skill in the art.

One aspect of the invention provides a method for identifying a candidate gene associated with a biological process including kidney function, renal toxicity, and/or kidney disorders comprising: a) using a gene expression level of at least one marker selected among Calbindin-D28k, KIM-1, OPN, EGF, Clusterin, VEGF, OAT-K1, Aldolase A, Aldolase B, Podocin, Alpha-2u and C4 as input for an algorithm for obtaining at least one numerical value I; and b) comparing the at least one numerical value I obtained in a) with a numerical value II obtained for the candidate gene. Preferably, the method further comprises step c), wherein the candidate gene is associated with the biological process if the value obtained in step b) correlates in a predetermined relationship to value II. In a particular embodiment of the invention, the predetermined relationship is 1 or greater. In another embodiment of the method, the predetermined relationship is 1 or less.

According to another particular embodiment, the gene expression level of at least one marker selected among Calbindin-D28k, KIM-1, OPN, EGF, Clusterin, VEGF, OAT-K1, Aldolase A, Aldolase B, Podocin, Alpha-2u and C4 is obtained from at least one body sample of an individual such as kidney tissue, blood or urine or from a kidney cell line. The at least one body sample is in a preferred embodiment two or more different body samples such as kidney tissue and blood. In a particularly preferred embodiment, the body sample or the cell line have been in contact with a cytotoxic agent. Preferably such cytotoxic agent is selected among cyclosporine, cisplatin, tacrolimus, aminoglycosides, sulfonamides and trimethadione.

In a particular embodiment of the invention, the method is a computer-executable method.

Antisense molecules

In another embodiment, activity of a target RNA (preferable mRNA) species, specifically its rate of translation, can be controllably inhibited by the controllable application of antisense nucleic acids. An "antisense" nucleic acid as used herein refers to a nucleic acid capable of hybridizing to a sequence-specific (e.g., non-poly A) portion of the target RNA, for example its translation initiation region, by virtue of some sequence complementarity to a coding and/or non-coding region. The antisense nucleic acids of the invention can be oligonucleotides that are double-stranded or single-stranded, RNA or DNA or a modification or derivative thereof, which can be directly administered in a controllable manner to a cell or which can be produced intracellularly by transcription of exogenous, introduced sequences in controllable quantities sufficient to perturb translation of the target RNA.

Preferably, antisense nucleic acids are of at least six nucleotides and are preferably oligonucleotides (ranging from 6 to about 200 oligonucleotides).

As discussed above, antisense nucleotides can be delivered to cells which express the described genes *in vivo* by various techniques, e.g., injection directly into the kidney tissue site, entrapping the antisense nucleotide in a liposome, by administering modified antisense nucleotides which are targeted to the kidney cells by linking the antisense nucleotides to peptides or antibodies that specifically bind receptors or antigens expressed on the cell surface.

However, with the above-mentioned delivery methods, it may be difficult to attain intracellular concentrations sufficient to inhibit translation of endogenous mRNA. Accordingly, in an alternative embodiment, the nucleic acid comprising an antisense nucleotide sequence is placed under the transcriptional control of a promoter, i.e., a DNA sequence which is required to initiate transcription of the specific genes, to form an expression construct. The antisense nucleic acids of the invention are controllably expressed intracellularly by transcription from an exogenous sequence. If the expression is controlled to be at a high level, a saturating perturbation or modification results.

In conclusion, antisense nucleic acids can be routinely designed to target virtually any mRNA sequence including the marker genes (Calbindin-D28k, KIM-1, OPN, EGF, Clusterin VEGF, OAT-K1, Aldolase A, Aldolase B, Podocin, Alpha-2u and C4) cited in the present document, and a cell can be routinely transformed with or exposed to nucleic acids coding for such antisense sequences such that an effective and controllable or saturating amount of the antisense nucleic acid is expressed. Accordingly the translation of virtually any RNA species in a cell can be modified or perturbed.

Small Molecule Drugs or Ligands

In addition, the activities of marker proteins (Calbindin-D28k, KIM-1, OPN, EGF, Clusterin, VEGF, OAT-K1, Aldolase A, Aldolase B, Podocin, Alpha-2u and C4) can be modified or perturbed in a controlled or a saturating manner by exposure to exogenous drugs or ligands. Since the methods of this invention are often applied to testing or confirming the usefulness of various drugs to treat kidney disorders, drug exposure is an important method of modifying/perturbing cellular constituents, both mRNA's and expressed proteins.

In a preferable case, a drug is known that interacts with only one marker protein in the cell and alters the activity of only that one marker protein, either increasing or decreasing the activity. Graded exposure of a cell to varying amounts of that drug thereby causes graded perturbations of network models having that marker protein as an input. Saturating exposure causes saturating modification/perturbation.

Antibodies and Antagonists

The term "antagonist" refers to a molecule which, when bound to the protein encoded by the gene, inhibits its activity. Antagonists can include, but are not limited to, peptides, proteins, carbohydrates, and small molecules.

In a particularly useful embodiment, the antagonist is an antibody specific for the markers (Calbindin-D28k, KIM-1, OPN, EGF, Clusterin, VEGF, OAT-K1, Aldolase A, Aldolase B, Podocin, Alpha-2u and C4). The antibody alone may act as an effector of therapy or it may recruit other cells to actually effect cell killing.

Treatment Modalities

In the case of treatment with an antisense nucleotide, the method comprises administering a therapeutically effective amount of an isolated nucleic acid molecule comprising an antisense nucleotide sequence derived from at least one marker identified in Table 1 above wherein the antisense nucleotide has the ability to change the transcription/translation of the at least one gene.

In the case of treatment with an antagonist, the method comprises administering to a subject a therapeutically effective amount of an antagonist that inhibits or activates a protein encoded by at least one marker identified in Table 1 above.

A "therapeutically effective amount" of an isolated nucleic acid molecule comprising an antisense nucleotide, nucleotide sequence encoding a ribozyme, double-stranded RNA, or antagonist, refers to a sufficient amount of one of these therapeutic agents to treat renal disease or toxicity. The determination of a therapeutically effective amount is well within the capability of those skilled in the art. For any therapeutic, the therapeutically effective dose can be estimated initially either in cell culture assays, e.g., of neoplastic cells, or in animal models, usually rats, mice, rabbits, dogs or pigs. The animal model may also be used to determine the appropriate concentration range and route of administration. Such information can then be used to determine useful doses and routes for administration in humans.

Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between toxic and therapeutically effects is the therapeutic index, and it can be expressed as the ratio LD50/ED50. Antisense nucleotides, ribozymes, double-stranded RNAs and antagonists that exhibit large therapeutic indices are preferred. The data obtained from cell culture assays and animal studies is used in formulating a range of dosage for human use. The dosage contained in such compositions is preferably within a range of circulating concentrations that include the ED50 with little or no toxicity. The dosage varies within this range, depending upon the dosage form employed, sensitivity of the patient, and the route of administration.

The exact dosage will be determined by the practitioner, in light of factors related to the subject that requires treatment. Dosage and administration are adjusted to provide sufficient levels of the active moiety or to maintain the desired effect. Factors that may be taken into account include the severity of the disease state, general health of the subject, age, weight and gender of the subject, diet, time and frequency of administration, drug combination(s), reaction sensitivities, and tolerance/response to therapy.

Normal dosage amounts may vary form 0.1 to 100,000 micrograms, up to a total dosage of about 1 g, depending upon the route of administration. Guidance as to particular dosages and methods of delivery is provided in the literature and generally available to practitioners in the art. Those skilled in the art will employ different formulations for nucleotides than for antagonists.

For therapeutic applications, the antisense nucleotides, nucleotide sequences encoding ribozymes, double-stranded RNAs (whether entrapped in a liposome or contained in a viral vector) and antibodies are preferably administered as pharmaceutical compositions containing the therapeutic agent in combination with one or more pharmaceutically acceptable carriers. The compositions may be administered alone or in combination with at least one other agent, such as stabilizing compound, which may be administered in any sterile, biocompatible pharmaceutical carrier, including, but not limited to, saline, buffered saline, dextrose and water. The compositions may be administered to a patient alone or in combination with other agents, drugs or hormones.

The pharmaceutical compositions may be administered by a number of routes including, but not limited to, oral, intravenous, intramuscular, intra-articular, intra-arterial, intramedullary, intrathecal, intraventricular, transdermal, subcutaneous, intraperitoneal, intranasal, enteral, topical, sublingual, or rectal means. In addition to the active ingredient, these pharmaceutical compositions may contain suitable pharmaceutically acceptable carriers comprising excipients and auxiliaries which facilitate processing of the active compounds into preparations which can be used pharmaceutically.

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

References cited

All references cited herein are incorporated herein by reference in their entirety and for all purposes to the same extent as if each individual publication or patent or patent application was specifically and individually indicated to be incorporated by reference in its entirety for all purposes. In addition, all GenBank accession numbers cited herein are incorporated herein by reference in their entirety and for all purposes to the same extent as if each such number was specifically and individually indicated to be incorporated by reference in its entirety for all purposes.

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatus within the scope of the invention, in addition to those enumerated herein, will be apparent to those skilled in the art from the foregoing description and accompanying drawings. Such modifications and variations are intended to fall within the scope of the appended claims. The present invention is to be limited only by the terms of the appended claims, along with the full scope of equivalents to which such claims are entitled.

In practicing the present invention, many conventional techniques in molecular biology, microbiology, and recombinant DNA are used. These techniques are well known and are explained in, for example, Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods in Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively).

**Experiment 1**

Rat kidney samples were obtained from a 2-weeks *in vivo* study conducted in order to identify markers of toxicity and efficacy for immunosuppressants including cyclosporin A (CsA; Neoral®). CsA is the reference compound for immunosuppression in terms of clinical applications but also in terms of research model. CsA inhibits early events after T-cell activation, blocking the transcriptional activation of several cytokines.

The kidney, as the major target for toxicity, was studied and the RNA expression changes were monitored in all the groups using the high density DNA-array system from Affymetrix. The in-life part of the study was conducted as follows:

Experimental animals

| | |
|---|---|
| Animal species and strain: | Rats, Crl:WI (GLX/BRL/HAN) IGS BR. |
| Number of animals per group: | 6 males |
| Age: | 8 weeks (at start of dosing). |
| Body weight range: | 100 to 300 g (at start of dosing). |
| Room relative humidity: | Approximately 40 to 70% (target range). |
| Lighting cycle: | Fluorescent light for a 12-hour light/12-hour dark cycle. |
| Animal caging: | Animals were gang housed in groups of the same sex in type IV Macrolon cages on sterilized softwood particle bedding (manufactured by Rettenmaier & Söhne, Ellwangen-Holzmühle, Germany) under optimal hygienic conditions. |
| Food: | NAFAG, No. 890 pelleted standard diet from NAFAG, Gossau, SG, Switzerland *ad libitum* (batches were given in the on-line raw data), except overnight before blood sampling for clinical pathology. |
| Analysis of food: | Microbiological contaminants investigated by the supplier, chemical contaminants by supplier and RCC Ltd., Environmental Chemical/Pharmaceutical Analytics, Itingen, Switzerland. |
| Water: | Tap water from the local supply was available *ad libitum* from polyethylene bottles. |
| Analysis of water: | Chemical and bacteriological contaminants investigated periodically during the year by municipal authorities and RCC Ltd., Environmental Chemical/Pharmaceutical Analytics, Itingen, Switzerland for compliance with Swiss drinking water specifications. |

The CsA concentration applied in the study was:

Group: 1: Control; 2: Treatment.
Test item: Neoral ® -Sandimmun (CsA): Dosage (mg/kg): 5; Vol.-dos. (mUkg): 5.

After the treatment period, kidneys of rats were collected and total RNA was extracted. Total RNA was extracted from frozen kidneys using TRIzol reagent (Life Technologies) according to the manufacturer's instructions. Total RNA was quantified by the absorbance at $\lambda$ = 260 nm ($A_{260nm}$), and the purity was estimated by the ratio $A_{260nm}/A_{280nm}$. Integrity was checked by denaturing gel electrophoresis. RNA was stored at -80˚C until analysis.

Good quality total RNA was used to synthesize double-stranded cDNA using the Superscript Choice System (Life Technologies). The cDNA was then *in vitro* transcribed (MEGAscript™ T7 Kit, Ambion) to form biotin labeled cRNA. Next, 12 to 15 $\mu$g of labeled cRNA were hybridized to the probe arrays for 16 hours at 45˚C. Arrays were then washed according to the EukGE-WS2 protocol (Affymetrix), and stained with 10 $\mu$g/ml of streptavidin-phycoerythrin conjugate (Molecular Probes). The signal was antibody amplified with 2 mg/ml acetylated BSA (Life Technologies), 100 mM MES, 1 M [Na+], 0.05 % Tween 20, 0.005 % Antiofoam (Sigma), 0.1mg/ml goat IgG and 0.5 mg/ml biotinylated antibody and re-stained with the streptavidin solution. After washing, the arrays were scanned twice with the Gene Array® scanner (Affymetrix).

After mining of the genomics data and under the experimental conditions used in each of the groups, several genes were found to be differentially expressed > 2-fold on each of the probe arrays and were selected for Real-Time PCR confirmation. The GeneSpring™ software was used to compare the expression level in the treatment groups and to sort the genes using clustering algorithms. These calculations separate the genes according to their expression variations and group the genes sharing a similar variation pattern (hierarchical clustering, K-means clustering). It also compares the distribution of the expression level in a specified group to the overall distribution and calculates the probability for a given group to belong to the overall distribution. Genes for which expression changes correlated with the pathological grading were selected. The five gene markers Calbindin-D28k, KIM-1, OPN, EGF and Clusterin constitute part of the specific profile observed on the DNA-arrays after treatment with this agent.

The primer sequences listed in Table 2 have been used for real-time quantitative PCR analysis.

Table 2: Primer and probe sequences used for the real-time quantitative PCR analysis

| Gene description | Primer name | primer sequence |
|---|---|---|
| Kidney injury molecule 1 (KIM-1) | rKIM1.forward<br>rKIM1.reverse<br>rKIM1.probe | 5'- CAC TCC ACT TCT GTC TTG ATG CTC -3'<br>5'- GCA CGT CTC CTC CCT GCA -3'<br><br>FAM5'- TGT TCC TAA ACT CAC CCA CTG AGC TCT GAA TT -3'TAMRA |
| Calbindin-D28k | rCABP28.forward<br>rCABP28. reverse<br><br>rCABP28. probe | 5'-ACA CTG TTG GTT CAA GCT GGC-3'<br><br>5'-CTT GGA AAT ATA GGC ATA GTA TCA GAC AGA T-3'<br><br>FAM5'-TGG TGG CAA GGG AAG GTA GCC AGA-3'TAMRA |
| Osteopontin | rOSTEO.forward<br>rOSTEO.reverse<br>rOSTEO.probe | 5'-GAC AGT CAG GCG AGT TCC AAA-3'<br>5'- CTT GTC CTC ATG GCT GTG AAA C -3'<br><br>FAM5'- CCA GCC TGG AAC ATC AGA GCC ACG -3'TAMRA |
| Epidermal growth factor precursor | rEGFp.forward<br>rEGFp.reverse<br>rEGFp.probe | 5'- GCA CGA CAT CAC TGT GGT GTC -3'<br>5'- ATC CCC AAG AGG AGC AGC A -3'<br>FAM5'- TCT GTG TGG TGG CGC TGG CC -3'TAMRA |
| Clusterin | rTRPM2.forward<br>rTRPM2.reverse<br>rTRPM2.probe | 5'- AAG GAG GGA ATC TCC CAG CTT -3'<br>5'- GCG CTG GAG ACA TGT GGA GT-3'<br><br>FAM5'- CCG AGG TTG CTG CAG ACC CCT AGA-3'TAMRA |

(continued)

| Gene description | Primer name | primer sequence |
|---|---|---|
| Alpha-2u (Lipocalin 2) | rLPC2.forward<br>r LPC2.reverse<br>r LPC2.probe | 5'- GGT CGG TGG GAA CAG AGA AA-3'<br>5'- AAG GAG CGA TTC GTC AGC TTT-3'<br><br>FAM5'- TGT TGT TAT CCT TGA GGC CCA GAG ACT TGG-3'TAMRA |

Clusterin may be of particular interest as a marker since the product of this gene is a secreted protein. The Clusterin protein level was indeed increased as confirmed by Western Blot analysis of serum samples of these animals after treatment with non-nephrotoxic compound (A), and three nephrotoxic compounds (B, C, D; Table 3).

Table 3: Measurements by Western Blotting of Clusterin protein serum levels after treatment with compounds (A, B, C, D).

| Treatment | Treated / Control mean (%)* | CV (%) |
|---|---|---|
| Control | 100 | 11.5 |
| Compound A | 122 | 16.0 |
| Compound B | 92 | 21.0 |
| Compound C | 127 | 20.0 |
| Compound D | 116 | 28.0 |

$$* = \frac{mean\ treated\ sample\ band\ volume}{mean\ control\ band\ volume} \times 100$$

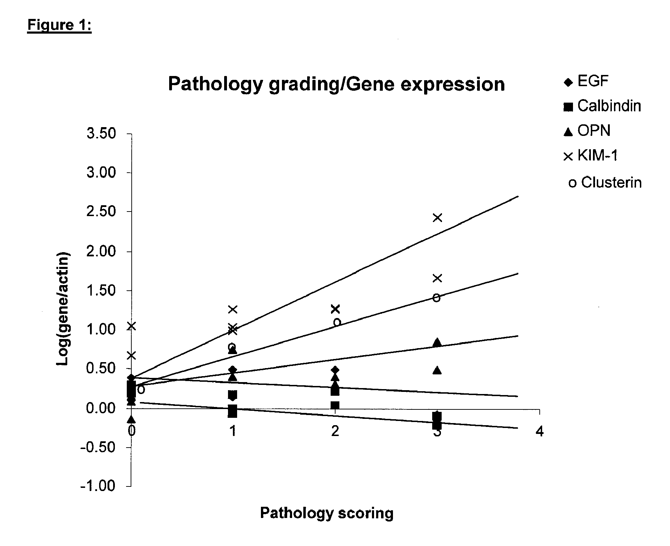

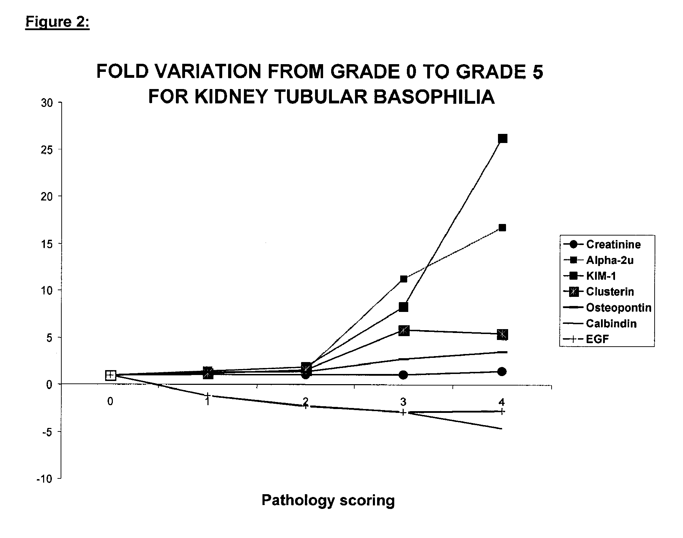

Figure 1 represents the evolution of the expression changes (Fold variation) of the gene markers (Calbindin-D28k, KIM-1, OPN, EGF and Clusterin) linked to kidney tubular basophilia. For comparison the evolution of creatinine excretion (a classical marker) is shown on the Figure 2 in order to demonstrate that the new gene markers (Calbindin-D28k, KIM-1, OPN, EGF and Clusterin) described in the present document are more affected and therefore more tightly associated to renal toxicity and are consequently more relevant and valuable.

**Experiment 2:**

A nonclinical study was initiated to establish the toxicologic effects of a test compound (TC1) when administered to rats for 14 and 42 days. The study was designed to evaluate the potential renal toxicity of the test compound.

The test compound was administered orally by gavage as a solution in cyclosporine A (CsA; Neoral®) Placebo Microemulsion Preconcentrate to groups of IGS Wistar Hannover [Crl:WI(Glx/BRL/Han)IGS BR] rats (N=5/sex/group) at doses of 20mg/kg/day, 60mg/kg/day for 14 and at doses of 10mg/kg/day, 25mg/kg/day for 42 days. An additional group of rats (males) received vehicle (CsA (Neoral®)) Placebo Microemulsion Preconcentrate) at an equivalent dosing volume of 5 mL/kg and served as controls. At the initiation of dosing, the animals were approximately 8 weeks of age. Kidney samples were collected at the day of necropsy.

Were monitored by PCR the genes described in the present invention (Calbindin-D28k, KIM-1, OPN, EGF and Clusterin) that together or alone, were used as markers of renal toxicity and therefore allowed the evaluation of the nephrotoxicity of the test compound.

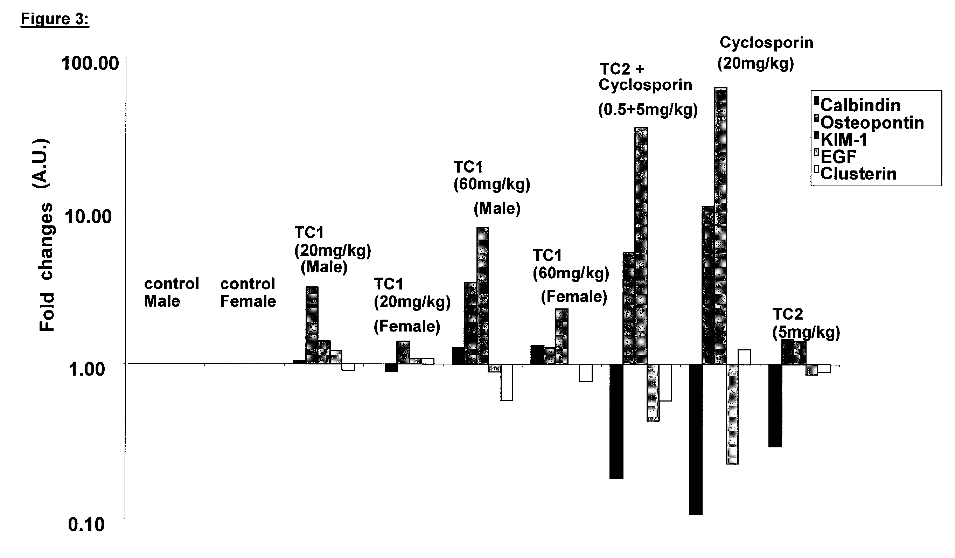

Based solely on the expression monitoring of the genes described in the present invention (Calbindin-D28k, KIM-1, OPN, EGF and Clusterin), it could be concluded prior to the pathological examination of kidney microscope slides that the test compound was less nephrotoxic than CsA (20 mg/kg/day), but slightly damaging for the kidney as demonstrated by the expression of the five gene makers. The kidney toxicity appeared however to be specific to the test compound after comparison with the CsA expression profile. Furthermore, after treatment for 42 days at the dose of 10 mg/kg/day, the gene expression profile for the genes described in the present invention (Calbindin-D28k, KIM-1, OPN, EGF and Clusterin) did not indicate marked kidney toxicity (Figure 3). In the figure the "fold-changes Vs control" represent the number of molecules for the genes described in the present invention (Calbindin-D28k, KIM-1, OPN, EGF and Clusterin) in the treated groups devided by the number of molecules for the genes described in the present invention (Calbindin-D28k, KIM-1, OPN, EGF and Clusterin) in the respective control groups.

These conclusions were confirmed at a later stage and proved the validity of the prediction made by the monitoring of the expression of the genes described in the present invention (Calbindin-D28k, KIM-1, OPN, EGF and Clusterin). The test compound renal toxicity was characterized as being tubular cytoplasmic vacuolation (which was different from the CsA-induced renal toxicity as predicted earlier by monitoring the genes described in the present invention (Calbindin-D28k, KIM-1, OPN, EGF and Clusterin).

**Experiment 3:**

Rats were treated once daily by oral gavage for 2 weeks with 5 or 20 mg/kg/day Cyclosporine A derivative (TC3). The kidneys were then harvested and the Podocin expression using primer sequences as listed in Table 4 measured by real-time quantitative PCR analysis. Table 5 shows the expression of Podocin gene in rats treated with TC3.

Table 4: Primer and probe sequences used for the real-time quantitative PCR analysis

| Podocin | rPODO.forward | 5'-CACTCTTCAGTCCTTGTCCACAGA-3' |
|---------|---------------|-------------------------------|
|         | rPODO.reverse | 5'- AAGGTTCAGCATGTCAAAGGGTAA-3' |
|         | rPODO.probe   | FAM5'-AGCCGTCCACCGTGGTTTTGCC-3'TAMRA |

Table 5: Measurements by PCR of Podocin mRNA levels

|  | Control | TC3 (5 mg/kg/day) | TC3 (20 mg/kg/day) |
|---|---------|-------------------|--------------------|
| Normalized expression | 65.766 | 64.500 | 35.917 |
| Standard deviation (STDEV) | 11.130 | 15.656 | 12.097 |

Rats display renal side effect similar to the ones observed after CsA treatment at a comparable dose, thus TC3 (20 mg/kg/day) being a nephrotoxic condition.

**Experiment 4:**

In an effort (a) to study the mechanism of drug-induced nephrotoxicity caused by cyclosporine A (CsA, Neoral®) and (b) to identify gene expression patterns that would identify specific genes (and their protein products) as potential early markers of nephrotoxicity, a 2-week rat study was performed using cyclosporine A. Male rats (Crl: Wist Han strain) were treated once daily by oral gavage for 2 weeks with either 5 or 20 mg/kg/day cyclosporine A. The kidneys were then harvested and total RNA extracted from frozen tissue using TRIzol reagent (Life Technologies) according to the manufacturer's instructions. Total RNA was quantified by the absorbance at $\lambda = 260$ nm ($A_{260nm}$) and the purity was estimated by the ratio $A_{260nm}/A_{280nm}$. Integrity was checked by denaturing gel electrophoresis. RNA was stored at -80°C until analysis. RNA was reverse transcribed using the Superscript Choice System (Life Technologies). The DNA was then *in vitro* transcribed (MEGAscript™ T7 Kit, Ambion) to form biotin labeled cRNA. Next, labeled cRNA was hybridized to the GeneChip™ probe arrays (rat array RU34A). Hybridization to the probe array, washing, staining and scanning was done according to the instructions of the manufacturer. RNA expression profiles were analyzed using Affymetrix RU34A rat gene chips.

Expression profile analysis was performed using the Compare and GeneChip Analysis programs. Compare analysis was performed using algorithm 1, average signals to average signals, listing genes that had a $\pm 2.5$-fold or higher ratio. Statistical analysis was performed using SigmaStat 2.03. All raw average difference values having negative values were adjusted to 1. For the genes listed in Table 6, the overall differences among the different treatment groups were statistically significant ($p < 0.001$).

Table 6: RNA expression profiles of KIM-1, OPN, Clusterin, Alpha-2u, C4, EGF Precursor, VEGF, OAT-K1, Aldolase A, Aldolase B measured by Affymetrix RU34A rat gene chips

| KIM-1 | Control | Cyclosporine A (5 mg/kg/day) | Cyclosporine A (20 mg/kg/day) |
|-------|---------|------------------------------|-------------------------------|
| *Average Difference Value* | 6.5 | 5.9 | 168 |

(continued)

| KIM-1 | Control | Cyclosporine A (5 mg/kg/day) | Cyclosporine A (20 mg/kg/day) |
|---|---|---|---|
| *Standard Error of the Mean* | 1.5 | 2 | 24 |
| *Number of samples tested* | 16 | 4 | 7 |
| | | | |
| OPN | Control | CsA (5 mg) | CsA (20 mg) |
| *Average Difference Value* | 705 | 1088 | 2730 |
| *Standard Error of the Mean* | 61 | 70 | 260 |
| *Number of samples tested* | 16 | 4 | 7 |
| | | | |
| Clusterin | Control | CsA (5 mg) | CsA (20 mg) |
| *Average Difference Value* | 305 | 302 | 2309 |
| *Standard Error of the Mean* | 12 | 26 | 198 |
| *Number of samples tested* | 16 | 4 | 7 |
| | | | |
| Alpha-2u | Control | CsA (5 mg) | CsA (20 mg) |
| *Average Difference Value* | 4.2 | 5.6 | 252 |
| *Standard Error of the Mean* | 2 | 4 | 53 |
| *Number of samples tested* | 16 | 4 | 7 |
| | | | |
| C4 | Control | CsA (5 mg) | CsA (20 mg) |
| *Average Difference Value* | 143 | 96 | 468 |
| *Standard Error of the Mean* | 13 | 22 | 74 |
| *Number of samples tested* | 16 | 4 | 7 |
| | | | |
| EGF | Control | CsA (5 mg) | CsA (20 mg) |
| *Average Difference Value* | 1007 | 1007 | 224 |
| *Standard Error of the Mean* | 63 | 187 | 34 |
| *Number of samples tested* | 16 | 4 | 7 |
| | | | |

(continued)

| VEGF | Control | CsA (5 mg) | CsA (20 mg) |
|---|---|---|---|
| *Average Difference Value* | 166 | 157 | 65 |
| *Standard Error of the Mean* | 10 | 16 | 4.9 |
| *Number of samples tested* | 16 | 4 | 7 |

| OAT-K1 | Control | CsA (5 mg) | CsA (20 mg) |
|---|---|---|---|
| *Average Difference Value* | 668 | 681 | 287 |
| *Standard Error of the Mean* | 51 | 39 | 38 |
| *Number of samples tested* | 16 | 4 | 7 |

| Aldolase A | Control | CsA (5 mg) | CsA (20 mg) |
|---|---|---|---|
| *Average Difference Value* | 192 | 211 | 7.3 |
| *Standard Error of the Mean* | 22 | 28 | 3.8 |
| *Number of samples tested* | 16 | 4 | 7 |

| Aldolase B | Control | CsA (5 mg) | CsA (20 mg) |
|---|---|---|---|
| *Average Difference Value* | 3222 | 2906 | 1523 |
| *Standard Error of the Mean* | 97 | 111 | 68 |
| *Number of samples tested* | 16 | 4 | 7 |

Genes upregulated by Cyclosporine A (CsA) treatment

Kidney Injury Molecule-1 (KIM-1)

One gene found to be significantly upregulated by the 20 mg/kg/day CsA treatment was Kidney Injury Molecule-1 (KIM-1) (probe set AF035963_at). As shown in Table 6, the expression of KIM-1 was induced 26-fold in rats treated with 20 mg/kg/day CsA as compared to the control rats (p<0.001). No induction of KIM-1 was detected in rats treated with 5 mg/kg/day CsA. The changes in KIM-1 expression by CsA (20 mg) compared to CsA (5 mg) are statistically significant (p<0.004).

Osteopontin (OPN)

A second gene found to be significantly upregulated by the 20 mg/kg/day CsA treatment was Osteopontin (OPN) (probe set M14656_at). As shown in Table 6, the expression of Osteopontin was induced 3.9-fold in rats treated with 20 mg/kg/day CsA as compared to the control rats (p<0.001). No induction of Osteopontin was detected in rats treated with 5 mg/kg/day CsA. The changes in Osteopontin expression by CsA (5 mg) compared to CsA (20 mg) are statistically significant (p<0.001).

Clusterin / Testosterone-repressed prostate message 2 (TRPM-2)

A third gene found to be significantly upregulated by the 20 mg/kg/day CsA treatment was Clusterin, also known as Testosterone-repressed prostate message 2 (TRPM-2) (probe set M64733mRNA_s_at). The expression of Clusterin was induced 7.6-fold in rats treated with 20 mg/kg/day CsA as compared to the control rats (Table 6; p<0.001). No induction of Clusterin was detected in rats treated with 5 mg/kg/day CsA. The changes in Clusterin expression by CsA (5 mg) compared to CsA (20 mg) are statistically significant (p<0.001).

Alpha-2u globulin related-protein (Alpha-2u)

A forth gene found to be significantly upregulated by the 20 mg/kg/day CsA treatment was Alpha-2u globulin related-protein (Alpha-2u) (probe set rc_AA946503_at), also known as Lipocalin 2 (LCN2) or Neutrophil Gelatinase-Associated Lipocalin (NGAL) in humans. The expression of Alpha-2u was induced 60-fold in rats treated with 20 mg/kg/day CsA as compared to the control rats (Table 6; p<0.001). No induction of Alpha-2u was detected in rats treated with 5 mg/kg/day CsA. The changes in Alpha-2u expression by CsA (20 mg) compared to Control and CsA (5 mg) are statistically significant (p<0.001).

Complement component 4 (C4)

A fifth gene found to be significantly upregulated by the 20 mg/kg/day CsA treatment was Complement component 4 (C4) (probe set U42719_at). C4 was induced 3.3-fold in rats treated with 20 mg/kg/day CsA as compared to the control rats (Table 6, p<0.001). No significant induction of C4 was detected in rats treated with 5 mg/kg/day CsA. The changes in C4 expression by CsA (5 mg) compared to CsA (20 mg) are statistically significant (p<0.001).

Genes downregulated by Cyclosporine A (CsA) treatment

Epidermal Growth Factor (EGF)

One gene found to be significantly downregulated by the 20 mg/kg/day CsA treatment was Epidermal Growth Factor (EGF) (probe set X12748cds_s_at). Rats treated with 20 mg/kg/day CsA showed 4.5-fold less EGF expression as compared to the control rats (Table 6, p<0.001). No significant changes in EGF expression were detected in rats treated with 5 mg/kg/day CsA The changes in EGF expression by CsA (20 mg) compared to CsA (5 mg) are statistically significant (p=0.004).

Vascular Endothelial Growth Factor (VEGF)

A second gene found to be significantly downregulated by the 20 mg/kg/day CsA treatment was Vascular Endothelial Growth Factor (VEGF) (probe set rc_AA850734_at). VEGF was repressed 2.6-fold in rats treated with 20 mg/kg/day CsA as compared to the control rats (Table 6, p<0.001). No significant repression of VEGF was detected in rats treated with 5 mg/kg/day CsA. The changes in VEGF expression by CsA (20 mg) compared to CsA (5 mg) are statistically significant (p<0.001).

Kidney-specific Organic Anion Transporter-K1 (OAT-K1)

A third gene found to be significantly downregulated by the 20 mg/kg/day CsA treatment was Kidney-specific Organic Anion Transporter-K1 (OAT-K1) (probe set D79981_at), also known as solute carrier family 21 member a4 (SLC21A4). Expression of OAT-K1 was repressed 2.3-fold in rats treated with 20 mg/kg/day CsA as compared to the control rats (Table 6, p<0.001). No significant changes in OAT-K1 expression were detected in rats treated with 5 mg/kg/day CsA. The changes in OAT-K1 expression by CsA (20 mg) compared to CsA (5 mg) are statistically significant (p<=0.019).

Aldolase A

A fourth gene found to be significantly downregulated by the 20 mg/kg/day CsA treatment was Aldolase A (probe set U20643_at). Aldolase A expression was repressed 26-fold in rats treated with 20 mg/kg/day CsA as compared to the control rats (Table 6; p<0.001). No significant changes in Aldolase A expression were detected in rats treated with 5 mg/kg/day CsA. The changes in Aldolase A expression by CsA (20 mg) compared to CsA (5 mg) are statistically significant (p<=0.042).

Aldolase B

A fifth gene found to be significantly downregulated by the 20 mg/kg/day CsA treatment was Aldolase B (probe set X02284_at). Aldolase B expression was repressed 2.1-fold in rats treated with 20 mg/kg/day CsA as compared to the control rats (Table 6; p<0.001). No significant changes in OAT-K1 expression were detected in rats treated with the 5 mg/kg/day CsA. The changes in Aldolase B expression by CsA (20 mg) compared to CsA (5 mg) are statistically significant (p<0.001).

**Claims**

1. An *in vitro* method for determining renal toxicity in an individual, comprising the steps of:

    (a) obtaining a body sample from said individual,
    (b) determining from the body sample the level of gene expression corresponding to the genes for Kidney Injury Molecule 1 (K1M-1) and Clusterin, to obtain a first set of values, and
    (c) comparing the first set of values with a second set of values corresponding to the level of gene expression assessed for KIM-1 and Clusterin under similar condition to that for step b) in a body sample of an individual not subject to renal toxicity, wherein the first value greater than the second value for KIM-1 and Clusterin gene expression is an indication that the individual is having, developing or sensitive to renal toxicity.

2. The method of claim 1, wherein in steps b) and c), the first set of values and second set of values respectively further includes the level of gene expression for the osteopontin (OPN) gene, wherein the first value greater than the second value for OPN gene expression is an indication that the individual is having, developing or sensitive to renal toxicity.

3. The method of claim 2, wherein in steps b) and c), the first set of values and second set of values respectively further includes the level of gene expression for one or more genes selected from the group consisting of the Calbindin-D28k, EGF, Alpha-2u globulin related-protein (Alpha-2u), Complement component 4 (C4), Vascular Endothelial Growth Factor (VEGF), Kidney-specific Organic Anion Transporter-K1 (OAT-K1), Aldolase A, Aldolase B and Podocin,
wherein the first value lower than the second value for Calbindin-D28K, EGF, VEGF, OAT-K1, Aldolase A, Aldolase B and/or Podocin gene expression is an indication that the individual of step a) is having, developing or sensitive to renal toxicity, and
wherein the first value greater than the second value for Alpha-2u and/or C4 gene expression is an indication that the individual is having, developing or sensitive to renal toxicity.

4. The method of any one of claims 1 to 3, wherein the gene expression levels as measured in mRNA expression levels in the body sample of the individual of step b), of Calbindin-D28k below 5.30E+08, of KIM-1 above 1.50E+07, of EGF below 2. 80E+08, of Osteopontin above 1.40E+08, of Clusterin above 1.90E+09, and/or Podocin below 3.00E+06, indicates that such individual is having, developing or sensitive to renal toxicity, wherein mRNA expression levels are expressed in absolute value.

5. The method of any one of claims 1 to 3, wherein a repression of gene expression of at least 4-fold for EGF, of at least 2-fold for VEGF, of at least 2-fold for OAT-K1, of at least 20-fold for Aldolase A, and/or for Aldolase B of at least 2-fold, and/or
wherein an induction of at least 20-fold for KIM-1, of at least 3-fold for OPN, of at least 7-fold for Clusterin, of at least 50-fold for Alpha-2u and/or for C4 of at least 3-fold is an indication that such individual is having, developing or sensitive to renal toxicity.

6. The method of claim 1, wherein the individual is under treatment with an agent suspected of being a cytotoxic agent.

7. The method of claim 6, wherein the cytotoxic agent is selected among cyclosporine, cisplatin, tacrolimus, aminoglycosides, sulfonamides and trimethadione.

<u>**Figure 1:**</u>

**Pathology grading/Gene expression**

◆ EGF
■ Calbindin
▲ OPN
× KIM-1
o Clusterin

Log(gene/actin)

Pathology scoring

**Figure 2:**

FOLD VARIATION FROM GRADE 0 TO GRADE 5 FOR KIDNEY TUBULAR BASOPHILIA

Figure 3:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9918120 A

- WO 03033527 A

**Non-patent literature cited in the description**

- **LINDER MW.** *Clin Chem,* 1997, vol. 43 (2), 254-266
- **FEHER JJ.** *Am J Physiol,* 1983, vol. 44, C303-C307
- **RHOTEN WB et al.** *Anat Rec,* 1990, vol. 227, 145-151
- **BORKE JL et al.** *Am J Physiol (Renal Fluid Electrolyte Physiol,* 1989, vol. 257, F842-F849
- **ARMBRECHT HJ et al.** *Endocrinology,* 1989, vol. 125, 2950-2956
- **GRENET O et al.** *Biochem Pharmacol,* 1998, vol. 55 (7), 1131-1133
- **GRENET O et al.** *Biochem Pharmacol,* 2000, vol. 59 (3), 267-272
- **STEINER S et al.** *Biochem Pharmacol,* 1996, vol. 51 (3), 253-258
- **ICHIMURA T et al.** *J Biol Chem,* 1998, vol. 273, 4135-4142
- **REINHOLT FP et al.** *Proc Natl Acad Sci U.S.A.,* 1990, vol. 87, 4473-4475
- **WADA T et al.** *Circ Res,* 1999, vol. 84, 166-178
- **NODA M et al.** *Proc Natl Acad Sci U. S. A.,* 1990, vol. 87, 9995-9999
- **PERSY VP et al.** *Kidney Int,* 1999, vol. 56 (2), 601-611
- **KOHRI K et al.** *Biochem Biophys Res Commun,* 1992, vol. 184, 859-864
- **KOHRI K et al.** *J Biol Chem,* 1993, vol. 268, 15180-15184
- **PRICE PM et al.** *Am J Physiol,* 1995, vol. 268 (4), F664-670
- **LAHAYE DH et al.** *FEBS Lett,* 1999, vol. 446 (2-3), 256-260
- **WANG S AND ; HIRSCHBERG R.** *Nephrol Dial Transplant,* 1997, vol. 12 (8), 1560-1563
- **DI PAOLO S et al.** *Nephrol Dial Transplant,* 1997, vol. 12, 2687-2693
- **MORIN NJ et al.** *Am J Physiol,* 1992, vol. 263, F806-F811
- **DENG JT et al.** *Transplant Proc,* 1994, vol. 26 (5), 2842-2844
- **YANG CW et al.** *Kindey Int,* 2001, vol. 60, 847-857

- **JENNE DE ; TSCHOPP J.** *Trends Biochem Sci,* 1992, vol. 14, 154-159
- **HAN B.H et al.** *Nat Med,* 2001, vol. 7, 338-343
- **WONG P et al.** *J Biol Chem,* 1993, vol. 268, 5021-5031
- **WONG P et al.** *Eur J Biochem,* 1994, vol. 331, 917-925
- **YAMADA K et al.** *Kidney Int,* 2001, vol. 59 (1), 137-146
- **SILKENSEN JR et al.** *J Am Soc Nephrol,* 1997, vol. 8 (2), 302-305
- **DARBY LA et al.** *Exp Nephrol,* 1995, vol. 3 (4), 234-239
- **KJELDSEN L et al.** *J Biol Chem,* 1993, vol. 268, 10425-10432
- **BUNDGAARD JR et al.** *Biochem Biophys Res Commun,* 1994, vol. 202, 1468-1475
- **COWLAND JB ; BORREGAARD N.** *Genomics,* 1997, vol. 45, 17-23
- **ZEREGA B et al.** *Eur J Cell Biol,* 2000, vol. 79, 165-172
- **WELCH TR et al.** *Clin Immunol,* 2001, vol. 101, 366-370
- **HO A et al.** *Arthritis Rheum,* 2001, vol. 44, 2350-2357
- **BENJAMIN LE.** *Am J Pathol,* 2001, vol. 158, 1181-1184
- **SAITO H et al.** *J Biol Chem,* 1996, vol. 271, 20719-20725
- **KISHI H et al.** *Proc Natl Acad Sci U.S.A.,* 1987, vol. 84, 8623-8627
- **KREUDER J et al.** *N Engl J Med,* 1996, vol. 334, 1100-1104
- **CROSS NC et al.** *Cell,* 1988, vol. 53, 881-885
- **KRANHOLD JF et al.** *Science,* 1969, vol. 165, 402-403
- **MASS RE et al.** *Am J Med Sci,* 1966, vol. 251, 516-523
- **KOMATSUDA A et al.** *Ren Fail.,* 2003, vol. 25 (1), 87-93
- **MASSON J et al.** *Kidney Int Suppl,* 1991, vol. 32, S28-S32
- **LANGHAM RG et al.** *Transplantation,* 2001, vol. 72, 1826-1829

- **ORIJI GK.** *Prostaglandins Leukot Essent Fatty Acids,* 1999, vol. 61, 119-123
- **GONZALEZ-CORREA JA et al.** *Thromb Res,* 1996, vol. 81, 367-381
- **WATERMAN, M.S.** Introduction to Computational Biology: Maps, sequences and genomes. Chapman & Hall, 1995
- Current Protocols in Molecular Biology. 1997, vol. I, II, a
- **SAMBROOK et al.** Molecular Cloning: A Laboratory Manual. Cold Spring Harbor Laboratory Press, 1989
- DNA Cloning: A Practical Approach. 1985, vol. I and II
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL.** A Practical Guide to Molecular Cloning; the series, Methods in Enzymology. Academic Press, Inc, 1984
- Gene Transfer Vectors for Mammalian Cells. Cold Spring Harbor Laboratory, 1987
- Methods in Enzymology. vol. 154, 155